(19) Europäisches Patentamt
European Patent Office
Office européen des brevets

(11) EP 0 903 343 B1

(12) EUROPEAN PATENT SPECIFICATION

(45) Date of publication and mention of the grant of the patent:
**28.05.2003 Bulletin 2003/22**

(21) Application number: **98117153.1**

(22) Date of filing: **10.09.1998**

(51) Int Cl.[7]: **C07D 237/32**, C07D 239/88, C07D 265/22, C07D 401/04, C07D 239/96, C07D 243/08, C07D 473/10, A61K 31/495

# (54) Alpha-Substituted phenylpropionic acid derivative and medicine containing the same

Alfa-substituierte Phenylpropionsäurederivate und diese enthaltende Arzneimittel

Dérivés d'acide phénylpropionique substitué en alfa et médicament le contenant

(84) Designated Contracting States:
**BE CH DE ES FR GB IT LI NL SE**

(30) Priority: **19.09.1997 JP 25502597**

(43) Date of publication of application:
**24.03.1999 Bulletin 1999/12**

(73) Proprietor: **SSP Co., Ltd.**
**Chuo-ku, Tokyo (JP)**

(72) Inventors:
- **Nagao, Yoshihiro**
  **Narita-shi, Chiba (JP)**
- **Konno, Fujiko**
  **Inga-bun, Chiba (JP)**
- **Kotake, Jiro**
  **Ichikawa-shi, Chiba (JP)**
- **Ishii, Fumio**
  **Sendai-shi, Miyagi (JP)**
- **Honda, Haruyoshi**
  **Inba-gun, Chiba (JP)**
- **Sato, Susumu**
  **Narita-shi, Chiba (JP)**

(74) Representative: **Hartz, Nikolai F., Dr. et al**
**Wächtershäuser & Hartz,**
**Patentanwälte,**
**Tal 29**
**80331 München (DE)**

(56) References cited:
**EP-A- 0 779 279**
**WO-A-91/19702**
**WO-A-97/31907**

- **CHEMICAL ABSTRACTS, vol. 126, no. 9, 3 March 1997 Columbus, Ohio, US; abstract no. 117964d, page 589; XP002032261 & JP 08 325264 A (SUMITOMO)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 903 343 B1

## Description

BACKGROUND OF THE INVENTION

Field of the Invention:

The present invention relates to α-substituted phenylpropionic acid derivatives or salts thereof, which are excellent in the effect of lowering blood glucose and lipid, and medicines comprising such a compound as an active ingredient.

Description of the Background Art:

Non-insulin dependent diabetes mellitius (NIDDM) is a disease caused by insulin resistance in a target tissue of insulin and impaired insulin secretion from β cells of pancreas. Sulfonylureas and insulin, which are in wide use for treating NIDDM at present, are agents for mainly improving impaired insulin secretion. Of these, the sulfonylureas, which are oral drugs, have a strong antidiabetic effect based on both pancreatic action and extrapancreatic action, but often cause serious hypoglycemia. Therefore, care must be taken in using them.

In recent years, the importance of insulin resistance in NIDDM has come to be indicated, so that there has been a demand for development of a drug exhibiting an antidiabetic effect by lightening insulin resistance in a target tissue of insulin without stimulating insulin secretion. As compounds having such an effect, have been developed thiazolidine derivatives such as troglitazone and pioglitazone (Japanese Patent Application Laid-Open Nos. 22636/1980, 51189/1985 and 157522/1994, etc.). In addition, some thiazolidine derivatives having a like effect and a bicyclic lactam structure or cyclic urethane structure have also been reported [Japanese Patent Application (KOHYO) Nos. 502144/1994, 502145/1994 and 502146/1994 (through PCT route), etc.']. Further, a great number of derivatives having no thiazolidine ring have also been reported [for example, Japanese Patent Application Laid-Open No. 170478/1991, Japanese Patent Application (KOHYO) No. 508054/1993 (through PCT route), etc.]. Furthermore, arylpropionic acid derivatives have also been reported as antidiabetics (WO91/19702, Japanese Patent Application Laid-Open Nos. 325250/1996, 325263/1996 and 325264/1996, etc.).

WO 97/31907 discloses substituted 4-hydroxy-phenylalcanoic acid derivatives. EP-A-0 779 279 discloses bicyclic heterocycles obtained by reacting substituted carboxylic acids with amines. WO 91/19702 discloses 3-(phenyl, chroman-2-yl, benzofuran-5-yl or benzoxazole-5-yl)-2-(hydroxy or mercapto)propionic acid derivatives and analogs. Chemical Abstracts, vol. 126, no. 9, March 3, 1997, Columbus , Ohio, US, abstract no. 117964d, page 589 discloses the preparation of substituted 3-phenylpropionic or acrylic acid derivatives.

However, the blood glucose-lowering effect brought about by these compounds which lighten insulin resistance has been not yet sufficient.

On the other hand, hyperlipemia and obesity have been becoming a problem in modern times when satiation and lack of exercise have been chronic, and there is hence a demand for development of medicines for treating these diseases.

SUMMARY OF THE INVENTION

Accordingly, it is an object of the present invention to provide a novel compound excellent in the effect of lowering blood glucose and lipid.

With the foregoing circumstances in view, the present inventors have synthesized various kinds of compounds and carried out an extensive investigation as to their pharmacological effects. As a result, it has been found that α-substituted phenylpropionic acid derivatives represented by a general formula (1), which will be described subsequently, are excellent in the effect of lowering blood glucose and lipid and hence are useful in preventing or treating diabetes mellitus, hyperlipemia, obesity and the like, thus leading to completion of the present invention.

According to the present invention, there is thus provided an α-substituted phenylpropionic acid derivative represented by the following general formula (1):

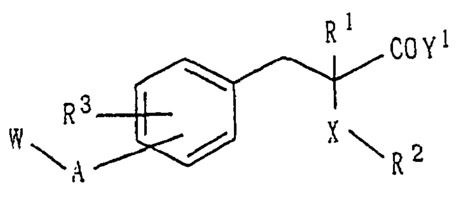

(1)

wherein W is a bicyclic lactam ring which may be substituted, A is an alkylene, alkyleneoxy or alkylenecarbonyl group which may be substituted by at least one hydroxy group, X is O, S, NH or $CH_2$, $Y^1$ is an amino, hydroxyamino, hydroxyalkylamino, monoalkylamino, dialkylamino, cyclic amino, hydroxy or lower alkoxy group, $R^1$ is a hydrogen atom, lower alkyl group, or $COY^2$ (in which $Y^2$ is an amino, hydroxyamino, hydroxyalkylamino, monoalkylamino, dialkylamino, cyclic amino, hydroxy or lower alkoxy group), $R^2$ is a lower alkyl, hydroxyalkyl, alkoxyalkyl or halogenoalkyl group, $COY^2$ (in which $Y^2$ has the same meaning as defined above), or a phenyl, pyridyl or aralkyl group which may be substituted, and $R^3$ is a hydrogen or halogen atom, or an alkyl, alkoxy, halogenoalkyl, amino, hydroxy or acyl group, or a salt thereof according to claim 1 or an α-substituted phenylpropionic acid derivative or a salt there of according to claim 2.

According to the present invention, there is also provided a medicine comprising the α-substituted phenylpropionic acid derivative or the salt thereof according to claim 1 or 2 as an active ingredient.

According to the present invention, there is further provided a medicinal composition comprising the α-substituted phenylpropionic acid derivative or the salt thereof according to claim 1 or 2 and a pharmaceutically acceptable carrier.

According to the present invention, there is still further provided use of the α-substituted phenylpropionic acid derivative represented by the general formula (1) or the salt thereof for the manufacture of a medicine.

The compounds (1) according to the present invention are excellent in the effect of lowering blood glucose and lipid and are hence useful as agents for preventing or treating diabetes mellitus, hyperlipemia, obesity and the like.

The above and other objects, features and advantages of the present invention will be readily appreciated as the same becomes better understood from the preferred embodiments of the present invention, which will be described subsequently in detail, and from the appended claims.

DETAILED DESCRIPTION OF THE PREFERRED EMBODIMENTS

The lactam ring represented by W in the α-substituted phenylpropionic acid derivatives according to the present invention, which are represented by the general formula (1), is selected from among groups represented by the following (W-1) to (W-6) and (W-9):

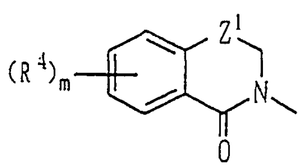
(W-1)

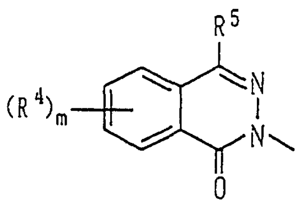
(W-2)

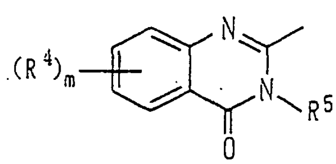
(W-3)

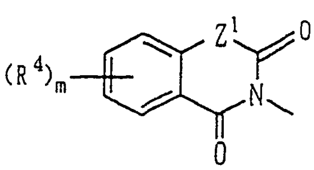
(W-4)

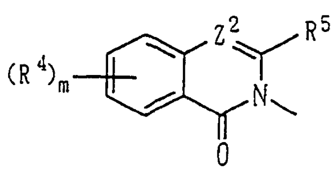
(W-5)

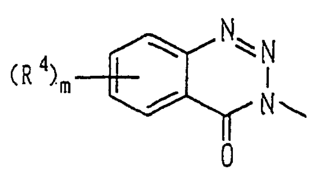
(W'-6)

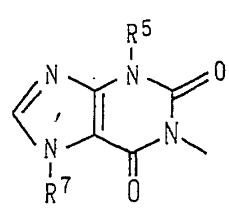
(W-9)

wherein $R^4$ is a hydrogen or halogen atom, an alkyl, alkoxy, halogenoalkyl, amino, hydroxy, cyano, carbamoyl, acyl, nitro, carboxy or sulfonamide group, or a phenyl or benzyloxy which may be substituted, $R^5$ is a hydrogen atom, an

alkyl group, or an aryl, aralkyl or pyridyl group which may be substituted, $R^6$ is a hydrogen atom or a lower alkyl group, $R^7$ is a lower alkyl, phenyl or aralkyl group, $Z^1$ is O, S, $CH_2$ or $NR^5$ (in which $R^5$ has the same meaning as defined above), $Z^2$ is N or CH, and m is an integer of 1 to 4.

In the above formulae, the alkyl groups represented by $R^4$ and $R^5$ are selected from linear or branched alkyl groups having 1 to 8 carbon atoms. Examples thereof include methyl, ethyl, n-propyl, isopropyl, n-butyl, sec-butyl, tert-butyl, pentyl, hexyl, heptyl and octyl groups. The alkoxy group represented by $R^4$ is selected from a linear or branched alkoxy group having 1 to 8 carbon atoms. Examples thereof include methoxy, ethoxy, propoxy, butoxy, pentyloxy, hexyloxy, heptyloxy and octyloxy groups. Examples of the halogen atom include fluorine, chlorine, bromine and iodine atoms. The halogenoalkyl group is selected from an alkyl group substituted by 1 to 3 halogen atoms and having 1 to 8 carbon atoms. Examples thereof include trifluoromethyl, trichloromethyl and tribromomethyl groups. The acyl group is selected from alkanoyl groups having 1 to 9 carbon atoms, such as formyl, acetyl and propionyl groups, and aroyl groups such as a benzoyl group. Examples of substituents on the phenyl or benzyloxy group include the alkyl groups, alkoxy groups, halogen atoms, halogenoalkyl groups and acyl group, which have been mentioned above, and besides amino, hydroxy, cyano, carbamoyl, nitro, carboxy, sulfonamide, phenyl and benzyloxy groups.

The lower alkyl groups represented by $R^6$ and $R^7$ are selected from linear or branched alkyl groups having 1 to 6 carbon atoms. Examples thereof include methyl, ethyl, n-propyl, isopropyl, n-butyl, isobutyl and sec-butyl groups. The aryl group represented by $R^5$ is selected from aryl groups having 6 to 14 carbon atoms, such as phenyl and naphthyl groups. The aralkyl groups represented by $R^5$ and $R^7$ are selected from phenyl-$C_{1-6}$-alkyl groups such as benzyl and phenetyl groups. Examples of substituents on the aryl, aralkyl or pyridyl group represented by $R^5$ include the same substituents as those mentioned in the phenyl group represented by $R^4$. The group W is particularly preferably (W-1), (W-2) (W-4) or (W-5).

The group A in the general formula (1) is a linear or branched alkylene group having 1 to 8 carbon atoms, which may be substituted by 1 to 5 hydroxy groups, a linear or branched alkyleneoxy group having 1 to 8 carbon atoms, which may be substituted by 1 to 5 hydroxy groups, or a linear or branched alkylenecarbonyl group having 2 to 9 carbon atoms, which may be substituted by 1 to 5 hydroxy groups. Specific examples of A include ethylene, trimethylene, propylene, tetramethylene, butylene, ethyleneoxy, trimethyleneoxy, 2-hydroxy-trimethyleneoxy, propyleneoxy, butyleneoxy, methylene-carbonyl, ethylenecarbonyl and trimethylenecarbonyl groups. Of these, the ethylene, trimethylene, ethyleneoxy and 2-hydroxytrimethyleneoxy groups are particularly preferred.

An atomic group represented by X is preferably an oxygen atom.

With respect to the groups represented by $Y^1$ and $Y^2$, the alkyl moieties of the mono- or dialkylamino groups or hydroxyalkylamino groups are linear or branched alkyl groups having 1 to 6 carbon atoms. Examples thereof include methyl, ethyl, n-propyl, isopropyl, n-butyl, isobutyl, sec-butyl and tert-butyl groups. The cyclic amino groups are selected from 4-membered to 7-membered cyclic amino groups such as piperazinyl, piperidinyl, pyrrolidinyl and azetidinyl groups. The alkoxy groups are selected from linear or branched alkoxy groups having 1 to 6 carbon atoms, such as methoxy, ethoxy, propoxy and butoxy groups.

The lower alkyl groups represented by $R^1$ and $R^2$ are selected from linear or branched alkyl groups having 1 to 6 carbon atoms, such as methyl, ethyl, n-propyl, isopropyl, n-butyl, isobutyl, sec-butyl and tert-butyl groups.

The hydroxyalkyl group represented by $R^2$ is selected from a hydroxy-$C_{1-6}$-alkyl group. Examples thereof include hydroxyethyl and hydroxypropyl groups. The alkoxyalkyl group represented by $R^2$ is selected from a $C_{1-6}$-alkoxy-$C_{1-6}$-alkyl group. Examples thereof include methoxyethyl, ethoxyethyl and methoxypropyl groups. The halogenoalkyl group represented by $R^2$ is selected from an alkyl group substituted by 1 to 3 halogen atoms and having 1 to 6 carbon atoms. Examples thereof include trifluoromethyl and trifluoroethyl groups.

The aralkyl group represented by $R^2$ is selected from phenyl-$C_{1-6}$-alkyl groups such as benzyl and phenetyl groups. Examples of substituents on the phenyl, pyridyl or aralkyl group represented by $R^2$ include alkyl groups, alkoxy groups, halogen atoms, halogenoalkyl groups, acyl groups, amino group, hydroxy group, cyano group, carbamoyl group, nitro group, carboxy group, sulfonamide group, phenyl group and benzyloxy group. $R^1$ is preferably a hydrogen atom. $R^2$ is preferably a lower alkyl group.

The alkyl group represented by $R^3$ is selected from a linear or branched alkyl group having 1 to 8 carbon atoms. Examples thereof include methyl, ethyl, n-propyl, isopropyl, n-butyl, sec-butyl, tert-butyl, pentyl, hexyl, heptyl and octyl groups. The alkoxy group represented by $R^3$ is selected from a linear or branched alkoxy group having 1 to 8 carbon atoms. Examples thereof include methoxy, ethoxy, propoxy, butoxy, pentyloxy, hexyloxy, heptyloxy and octyloxy groups. Examples of the halogen atom include fluorine, chlorine, bromine and iodine atoms. The halogenoalkyl group is selected from an alkyl group substituted by 1 to 3 halogen atoms and having 1 to 8 carbon atoms. Examples thereof include trifluoromethyl, trichloromethyl and tribromomethyl groups. The acyl group is selected from alkanoyl groups having 1 to 9 carbon atoms, such as formyl, acetyl and propionyl groups, and aroyl groups such as a benzoyl group. $R^3$ is preferably a hydrogen atom.

The compounds (1) according to the present invention according to claim 1 or 2 may include various kinds of pharmaceutically acceptable solvates such as hydrates, and those of polymorphism. Since the compounds (1) according to the present invention according to claim 1 or 2 have an asymmetric carbon atom, stereoisomers exist. All these isomers are included in the present invention.

No particular limitation is imposed on salts of the compounds (1) according to the present invention according to claim 1 or 2 so far as they are pharmaceutically acceptable salts. Preferable examples of such salts include hydrogen halide salts such as hydrofluorides, hydrochlorides, hydrobromides and hydriodides; inorganic acid salts such as carbonates, nitrates, perchlorates, sulfates and phosphates; lower alkyl-sulfonates such as methanesulfonates, ethanesulfonates and trifluoromethanesulfonates; arylsulfonates such as benzenesulfonates and p-toluene-sulfonates; organic acid salts such as fumarates, maleates, succinates, citrates, tartrates and oxalates; amino acid salts such as glutamates and aspartates; and salts with alkali and alkaline earth metals such as sodium, potassium and calcium.

The compounds (1) according to the present invention according to claim 1 or 2 can be prepared, for example, in accordance with any of the following Preparation Processes 1 to 8.

<Preparation Process 1>

Case of $R^1$ = H and X = O in the general formula (1):

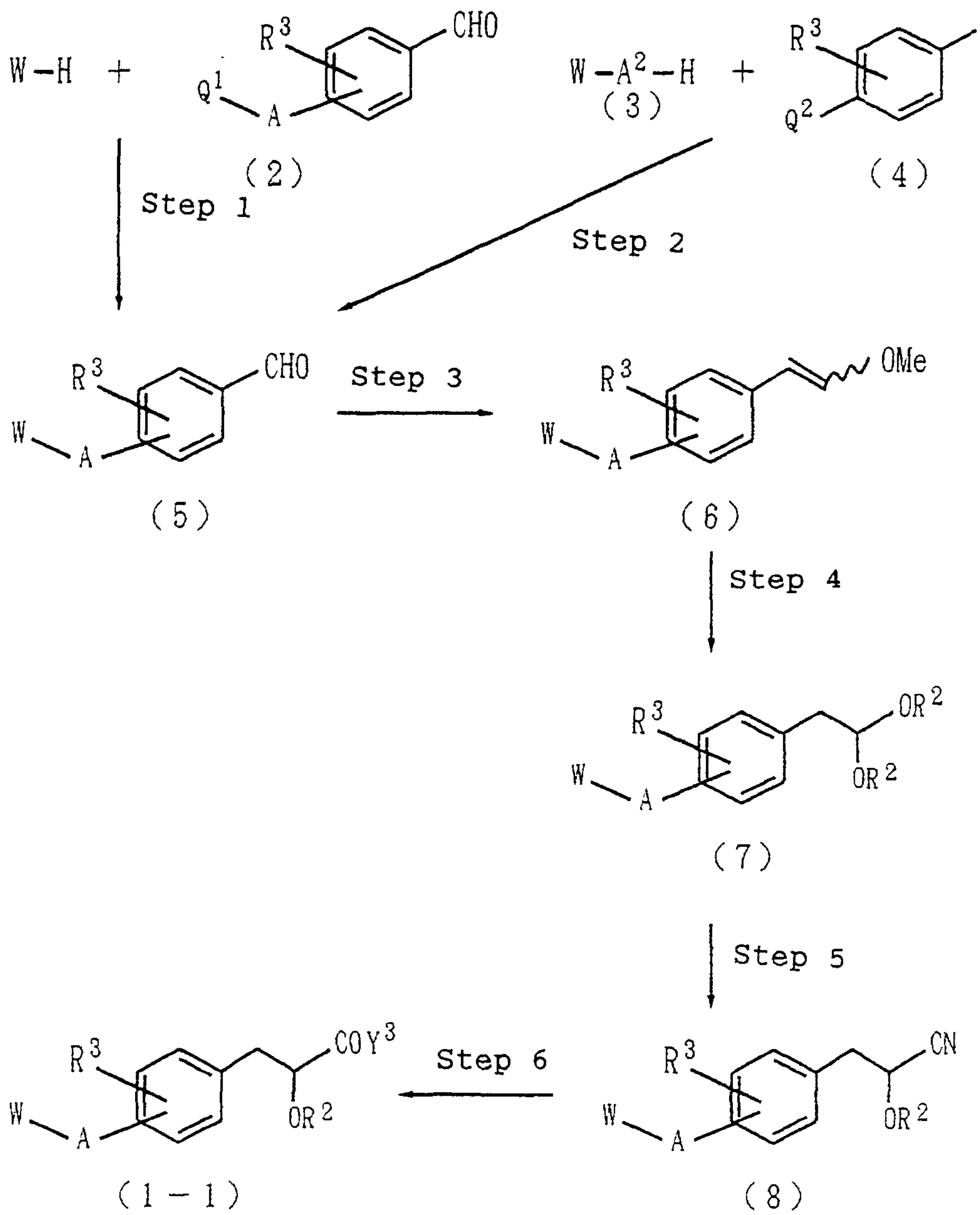


(2) (3) (4) (5) (6) (7) (8) (1 − 1)

wherein $R^2$, $R^3$, W and A have the same meanings as defined above, $A^2$ is an alkyleneoxy group which may be substituted by at least one hydroxyl group, $Y^3$ is an amino, hydroxyl or lower alkyl group, $Q^1$ is a leaving group, and $Q^2$ is a halogen atom.

More specifically, a compound (1-1) according to the present invention is prepared in the following manner. A compound represented by a general formula (2) is reacted with a compound represented by a general formula W-H to form a compound represented by a general formula (5) (Step 1). The 4-substitution product of compounds (5) may also be synthesized by the reaction of a compound represented by a general formula (3) with a compound represented by a general formula (4) (Step 2). The compound (5) is then subjected to a Wittig reaction with (methoxymethyl)-triphenylphosphonium chloride (Step 3), and the resultant compound represented by a general formula (6) is reacted with its corresponding alcohol to form an acetal derivative represented by a general formula (7) (Step 4). The acetal derivative is then reacted with trimethylsilyl nitrile to form a compound represented by a general formula (8) (Step 5). Finally, the compound (8) is hydrolyzed or reacted with an alcohol in the presence of an acid catalyst (Step 6), whereby the compound (1-1) according to the present invention can be prepared. The individual steps will hereinafter be described in detail.

Step 1:

The compound (2) is reacted with the compound W-H in the presence of proper base and solvent, whereby the compound (5) can be obtained.

The compound (2) can be prepared by halogenation or sulfonylation of the terminal hydroxy group in the benzaldehyde derivative substituted by a hydroxyalkoxy group or hydroxyalkyl group, which has been purchased as a commercially available reagent or synthesized in accordance with a known method [for example, a method described in Journal of Heterocyclic Chemistry, 6, 243 (1969) or Japanese Patent Application Laid-Open No. 92228/1996]. Examples of the leaving group ($Q^1$) thereof include halogen atoms such as chlorine, bromine and iodine atoms, and methanesulfonyloxy, p-toluenesulfonyloxy and trifluoromethanesulfonyloxy groups. Of these, the methanesulfonyloxy group is particularly preferred.

Examples of the base used in the reaction of the compound (2) with the compound W-H include sodium hydride, calcium hydride, potassium t-butoxide, sodium hydroxide, potassium hydroxide and potassium carbonate. No particular limitation is imposed on the solvent used herein so far as it does not affect the reaction. Examples of the solvent used include ethers such as tetrahydrofuran and dioxane; hydrocarbons such as benzene and toluene; amides such as dimethylformamide, dimethylacetamide and N-methyl-α-pyrrolidone; and sulfoxides such as dimethyl sulfoxide. The reaction is carried out in a temperature range of from a temperature under ice cooling to a reflux temperature under heating. It is particularly preferred to conduct the reaction by heating and stirring the reactants at 70 to 100°C for 2 to 5 hours in the presence of potassium carbonate in dimethylformamide.

Step 2:

The compound (3) which is a starting material can be prepared, for example, in accordance with the method described in Journal of Medicinal Chemistry, 11, 1038 (1968); or Journal of Medicinal Chemistry, 38, 130 (1995). This compound (3) is reacted with the p-halogeno-benzaldehyde derivative (4) in the presence of proper base and solvent, whereby the compound (5) can be obtained. Examples of the halogen atom $Q^2$ in the a p-halogeno-benzaldehyde derivative (4) include, fluorine, chlorine, bromine and iodine atoms, with the fluorine atom being particularly preferred. Examples of the base used in this reaction include sodium hydride, potassium t-butoxide, sodium hydroxide and potassium hydroxide. No particular limitation is imposed on the solvent used herein so far as it does not affect the reaction. Examples of the solvent used include ethers such as tetrahydrofuran and dioxane; hydrocarbons such as benzene and toluene; amides such as dimethylformamide, dimethylacetamide and N-methyl-α-pyrrolidone; and sulfoxides such as dimethyl sulfoxide. The reaction is carried out in a temperature range of from a temperature under ice cooling to a reflux temperature under heating. The reaction time is about 0.5 to 24 hours. It is particularly preferred to conduct the reaction by adding sodium hydride little by little to a dimethyl sulfoxide solution of a mixture of the compound (3) and the compound (4) under ice cooling and then stirring the resultant mixture for 1 to 3 hours at room temperature or so.

Step 3:

The compound (5) is subjected to the Wittig reaction with (methoxymethyl)triphenylphosphonium chloride, which is a commercially available reagent, in the presence of proper base and solvent, whereby the compound (6) can be obtained [the compound (6) is obtained as a mixture of E:Z]. Examples of the base used in this reaction include n-butyllithium, sec-butyllithium, lithium diisopropylamide, potassium t- butoxide and sodium methoxide. No particular limitation is imposed on the solvent used herein so far as it does not affect the reaction. Examples thereof include

ethers such as diethyl ether and tetrahydrofuran; hydrocarbons such as benzene and toluene; and alcohols such as ethanol. It is particularly preferred to conduct the reaction by preparing lithium diisopropylamide in a flask containing tetrahydrofuran and stirring the reaction mixture at a reaction temperature of from -10°C to 20°C for about 3 to 5 hours.

Step 4:

The compound (6) is reacted with its corresponding alcohol in the presence of an acid catalyst, whereby the compound (7) can be prepared.

When the alcohol has a low boiling point, the alcohol itself is used as a solvent. When the alcohol has a high boiling point on the other hand, a hydrocarbon such as benzene or toluene, or an amide such as dimethylformamide or dimethylacetamide may be used as a solvent. Examples of the acid catalyst include p-toluenesulfonic acid and methanesulfonic acid. The reaction is carried out in a temperature range of from a warming temperature to a reflux temperature under heating. The reaction time is about 1 to 24 hours.

Step 5:

The compound (7) is reacted with an excess amount of trimethylsilyl nitrile in the presence of a boron trifluoride etherate catalyst in methylene chloride, whereby the compound (8) can be prepared. The reaction is carried out at a temperature of from 0 to 20°C for about 0.5 to 2 hours.

Step 6:

In general, the compound (8) is hydrolyzed under basic conditions, whereby the compound (1-1) according to the present invention can be prepared. Examples of the base used include sodium hydroxide and potassium hydroxide. As a solvent for the reaction, is used a mixed solvent of ethanol-water, dioxane-water or the like. The reaction is carried out in a temperature range of from 80°C to a reflux temperature for about 0.5 to 5 hours. An ester derivative corresponding to the compound (1-1) can be prepared by using a compound in which $Y^3$ in the compound (1-1) according to the present invention is $NH_2$, and heating this compound and an alcohol with stirring in the presence of a catalytic amount of a Lewis acid such as titanium tetrachloride in 1N hydrochloric acid.

<Preparation Process 2> Case of $R^1$ = H and X = O in the general formula (1):

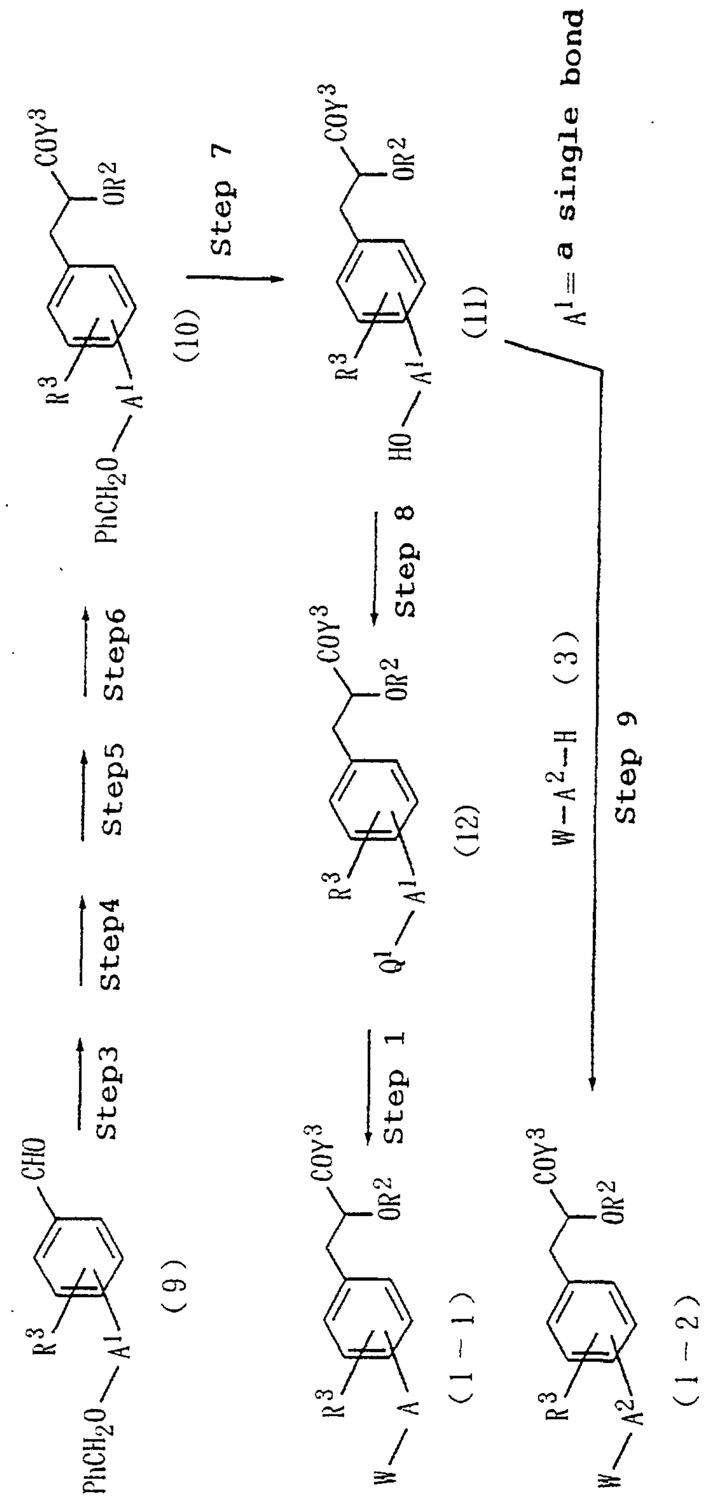


wherein W, $R^2$, $R^3$, $Y^3$, A, $A^2$ and $Q^1$ have the same meanings as defined above, and $A^1$ means A or a single bond.

More specifically, a benzaldehyde derivative represented by a general formula (9) is allowed to react in the same manner as in Step 3 to Step 6 described in Preparation Process 1 to form a compound represented by a general formula (10). The benzyl group which is a protecting group is then removed from the compound (10) to obtain a compound represented by a general formula (11) (Step 7). After the terminal hydroxy group of this compound is converted into a leaving group ($Q^1$) (Step 8), the resultant compound is allowed to react in the same manner as in Step 1 of Preparation Process 1, whereby the compound (1-1) according to the present invention can be prepared. When $A^1$ in the compound represented by the general formula (11) is a single bond, the compound is subjected to a condensation reaction with a compound represented by the general formula (3), whereby a compound (1-2) according to the present invention can be obtained (step 9). The individual steps will hereinafter be described.

Step 7:

The compound (9), which is a starting material, is purchased as a commercially available reagent or synthesized in accordance with a known method [for example, a method described in Journal of Heterocyclic Chemistry, 6, 243 (1969) or Japanese Patent Application Laid-Open No. 92228/1996], and is converted into a compound of the general formula (10) in the same manner as in Step 3 to Step 6 described in Preparation Process 1. This compound (10) is reduced by catalytical hydrogenation, whereby the compound of the general formula (11) can be prepared. Examples of the catalyst used in this reaction include palladium catalysts such as palladium on charcoal, palladium black and palladium hydroxide; platinum catalysts such as platinum oxide and platinum black; and nickel catalysts such as Raney nickel. No particular limitation is imposed on a solvent used so far as it does not affect the reaction. Examples thereof include methanol, ethanol, dioxane, dimethylformamide, acetic acid and a mixed solvent of ethanol-acetic acid. The reaction is carried out at atmospheric pressure or under pressure at room temperature or under heating at about 60 to 100°C.

Step 8:

The compound (12) is prepared by halogenating or sulfonylating the terminal hydroxy group of the phenylpropionic acid derivative (11), the benzene ring of which has been substituted by a hydroxyalkoxy group, hydroxyalkyl group or hydroxyalkanoyl group, in the presence or absence of a base and a solvent. Examples of the leaving group ($Q^1$) thereof include halogen atoms such as chlorine, bromine and iodine atoms, and methanesulfonyloxy, p-toluenesulfonyloxy and trifluoromethanesulfonyloxy groups. Of these, the methanesulfonyloxy group is particularly preferred. No particular limitation is imposed on the solvent used in the reaction so far as it does not affect the reaction. Examples of the solvent used include chlorinated hydrocarbons such as methylene chloride and chloroform; ethers such as tetrahydrofuran and dioxane; hydrocarbons such as benzene and toluene; amides such as dimethylformamide, dimethylacetamide and N-methyl-α-pyrrolidone; and sulfoxides such as dimethyl sulfoxide. Examples of the base used include triethylamine, pyridine, 4-dimethyl-aminopyridine, sodium hydride, sodium hydroxide and sodium carbonate.

The compound (12) thus obtained can be converted into the compound (1-1) according to the present invention in the same manner as in Step 1 of Preparation Process 1.

Step 9:

Among the compounds represented by the general formula (11), a compound in which $A^1$ is a single bond can be converted into the compound (1-2) according to the present invention by a Mitsunobu reaction (see Organic Reaction, 42, 335) with a compound represented by the general formula (3), which has been described in Preparation Process 1. More specifically, each 1 to 3 equivalents of triphenylphosphine and dialkyl (dimethyl, diethyl or diisopropyl) azobiscarboxylate are added to a solution of the compound (3) and compond (11) in methylene chloride, tetrahydrofuran, benzene, toluene, ether, dioxane or dimethylformamide, and the reaction is conducted in a temperature range of from -5°C to a reflux temperature under heating for about 1 to 24 hours, whereby the compound (1-2) according to the present invention can be obtained.

<Preparation Process 3> Case of $R^1$ = H in the general formula (1):

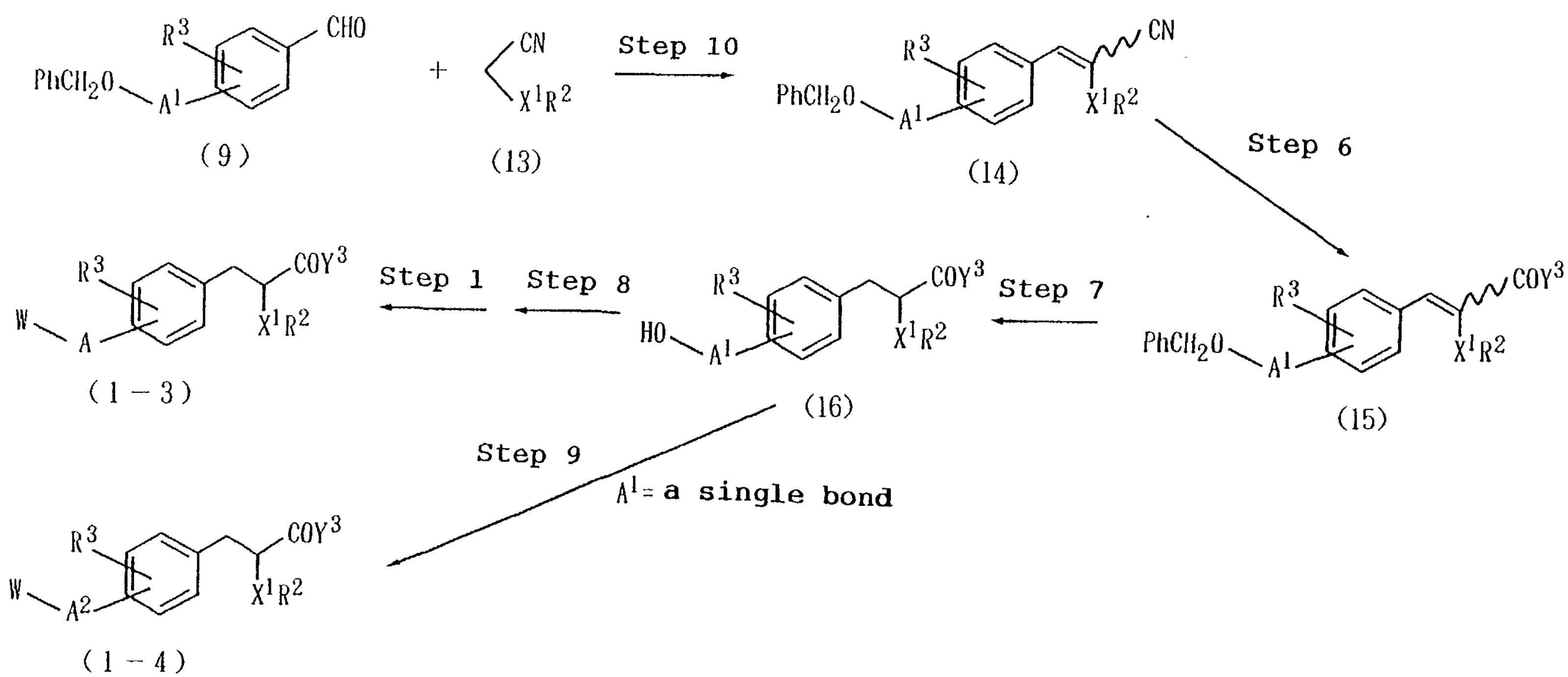


(9) (13) (14) (15) (16) (1 − 3) (1 − 4)

wherein W, A, $R^2$, $R^3$, $A^1$, $A^2$ and $Y^3$ have the same meanings as defined above, and $X^1$ is O, S or $CH_2$.

A compound represented by the general formula (9) is condensed with a compound represented by a general formula (13) to form a compound represented by a general formula (14) (Step 10). The compound thus obtained is allowed to react in the same manner as in Step 6 and Step 7 of Preparation Process 2, whereby a compound represented by a general formula (16) can be obtained. Subsequently, the compound (16) can be converted into a compound (1-3) according to the present invention in the same manner as in Step 8 and Step 1 of Preparation Process 2. When $A^1$ in the compound represented by the general formula (16) is a single bond, the compound is subjected to a Mitsunobu reaction in the same manner as in Step 9 of Preparation Process 2, whereby a compound (1-4) according to the present invention can be obtained. Step 10 will hereinafter be described in detail.

Step 10:

The compound (13), which is a starting material, can be purchased as a commercially available reagent or prepared in accordance with a known method [for example, a method described in Organic Synthesis Collect, Volume II, 387, Japanese Patent Application Laid-Open No. 136391/1994, or the like]. This compound is condensed with the compound represented by the general formula (9) in the presence of a proper base or catalyst, whereby the compound (a mixture of E:Z) of the general formula (14) can be prepared. Examples of the base used in the reaction include sodium hydride, potassium t-butoxide and pyridine. When the catalyst is used, piperidine and acetic acid, piperidinium acetate, piperidinium benzoate, or the like may be used. No particular limitation is imposed on a solvent used so far as it does not affect the reaction. Examples of the solvent used include ethers such as tetrahydrofuran and dioxane; hydrocarbons such as benzene and toluene; amides such as dimethylformamide, dimethylacetamide and N-methyl-$\alpha$-pyrrolidone; and sulfoxides such as dimethyl sulfoxide.

<Preparation Process 4> Case of $R^1$ = H in the general formula (1):

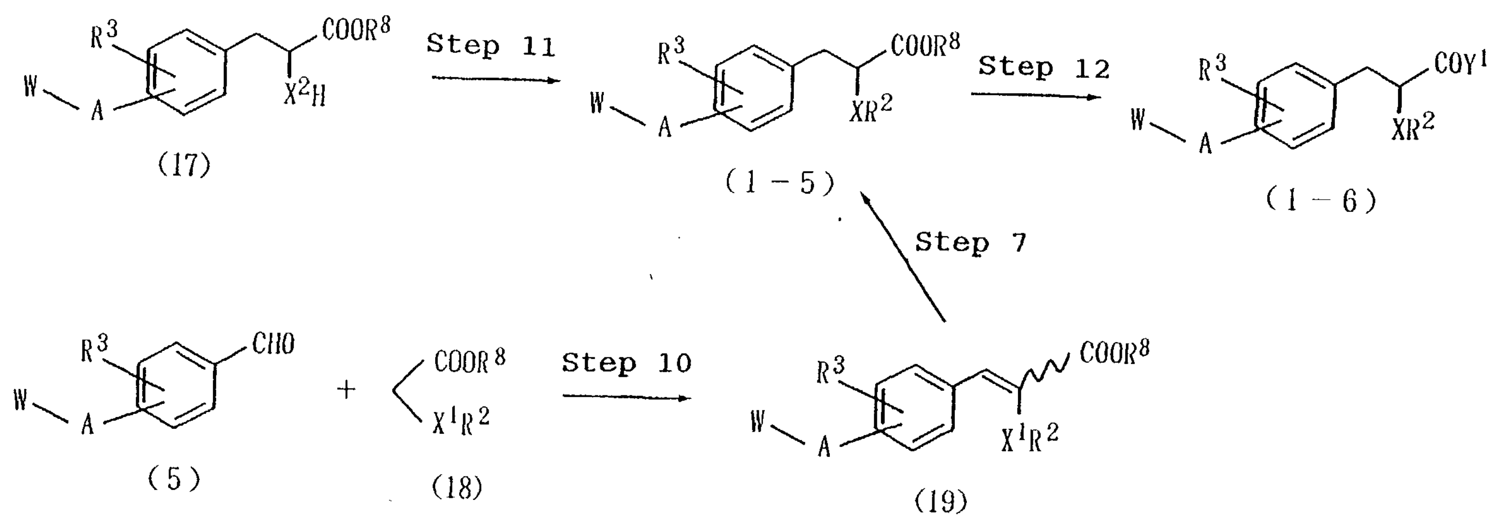


(17) (1 - 5) (1 - 6) (5) (18) (19)

wherein W, A, $Y^1$, $R^2$, $R^3$, $X^1$ and $A^2$ have the same meanings as defined above, $R^8$ is a lower alkyl group, and $X^2$ is O or NH.

More specifically, a compound represented by a general formula (17) is converted into a compound (1-5) according to the present invention by an ordinary alkylating method (Step 11). Subsequently, this compound is hydrolyzed or reacted with any of various amines, whereby a compound (1-6) according to the present invention can be prepared (Step 12). Alternatively, the compound (5) and a compound (18) are successively treated in the same manner as in Step 10 of Preparation Process 3 and Step 7 of Preparation Process 2, whereby the compound (1-5) according to the present invention can also be obtained. The individual steps will hereinafter be described.

Step 11:

The compound represented by the general formula (17), which is a starting material, can be synthesized in accordance with a known method [for example, a method described in Organic Synthesis Collect, Volume III, 586 (1955); Journal of Chemical Society, 1808 (1951); Synthesis, 793 (1992); Japanese Patent Application Laid-Open No. 325263/1996; or the like]. This compound or a salt thereof is reacted with a halide (iodide, bromide or chloride), whereby the compound (1-5) according to the present invention can be obtained. This reaction is carried out in the presence of a base in a proper solvent. Examples of the solvent used include dimethylformamide, dimethyl sulfoxide, methanol, ethanol, ethoxyethanol, tetrahydrofuran, dioxane and acetonitrile. Examples of the base used include triethylamine, N,N-diisopropylethylamine, pyridine, sodium hydroxide, potassium hydroxide, potassium carbonate, sodium hydrogencarbonate, sodium hydride and potassium hydride. The reaction is performed at room temperature or under heating with stirring.

Step 12:

The compound (1-5) according to the present invention is hydrolyzed in the presence of proper solvent and base, whereby the compound (1-6) according to the present invention can be obtained as its corresponding carboxylic acid. Alternatively, the compound (1-5) according to the present invention is reacted with any of various amines in the presence or absence of a solvent, or the above carboxylic acid is converted into an active derivative (for example, an acid halide or mixed acid anhydride) and then reacted with any of various amines, whereby the compound (1-6) according to the present invention can be prepared. With respect to the solvent and base used in the hydrolysis, the same conditions as those in Step 6 of Preparation Process 1 may be used.

<Preparation Process 5>

Case of $R^1$ = H and X = S in the general formula (1):

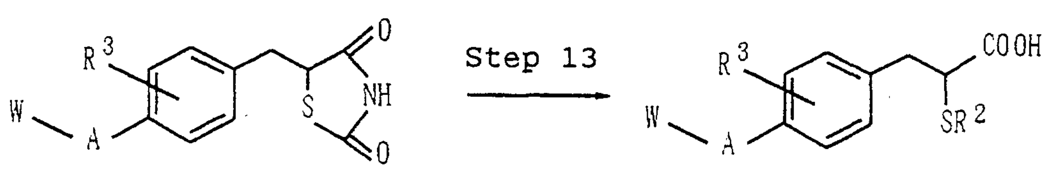


(20) (1 - 7)

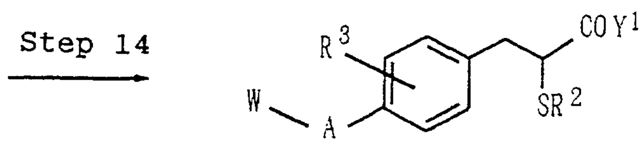


(1 - 8)

wherein W, A, $R^2$, $R^3$ and $Y^1$ have the same meaning as defined above.

More specifically, a compound represented by a general formula (20) is hydrolyzed under basic conditions and then reacted with its corresponding halide, whereby a compound (1-7) according to the present invention (Step 13). This compound is further converted into an active carboxylic acid derivative and then reacted with any of various amines and alcohols, whereby a compound (1-8) according to the present invention can be prepared (Step 14). The individual steps will hereinafter be described.

Step 13:

The compound represented by the general formula (20) (EP 0787727-A1), which is a starting material, is hydrolyzed under basic conditions and then reacted with its corresponding halide, whereby the compound (1-7) according to the present invention can be prepared. Specifically, after the compound of the general formula (20) is gently heated to reflux for 0.5 to 1 hour in a 15% aqueous solution of sodium hydroxide, a methanol solution of the corresponding halide (iodide, bromide or chloride) is added at room temperature, and the mixture is stirred for about 1 to 3 hours, whereby the compound (1-7) according to the present invention can be obtained.

Step 14:

The carboxylic acid, which is the compound (1-7) according to the present invention, is converted into an active derivative (for example, an acid halide or mixed acid anhydride) and then reacted with any of various amines and alcohols, or the compound (1-7) according to the present invention is reacted with any of various amines and alcohols in the presence of a proper condensation agent, whereby the compound (1-8) according to the present invention can be prepared.

Examples of the condensation agent used include carbonyldiimidazole, 1-hydroxy-2(1H)-pyridone, N-hydroxy-succinimide, diphenylphosphorylazide, N,N-dicyclohexyl-carbodiimide and 1-ethyl-3-(3-dimethylaminopropyl)-carbodiimide hydrochloride. The reaction is carried out in the presence of a proper base, for example, an organic base such as triethylamine or pyridine, according to the kind of the condensation agent used.

<Preparation Process 6> Case of X = O and $R^1$ = alkyl in the general formula (1):

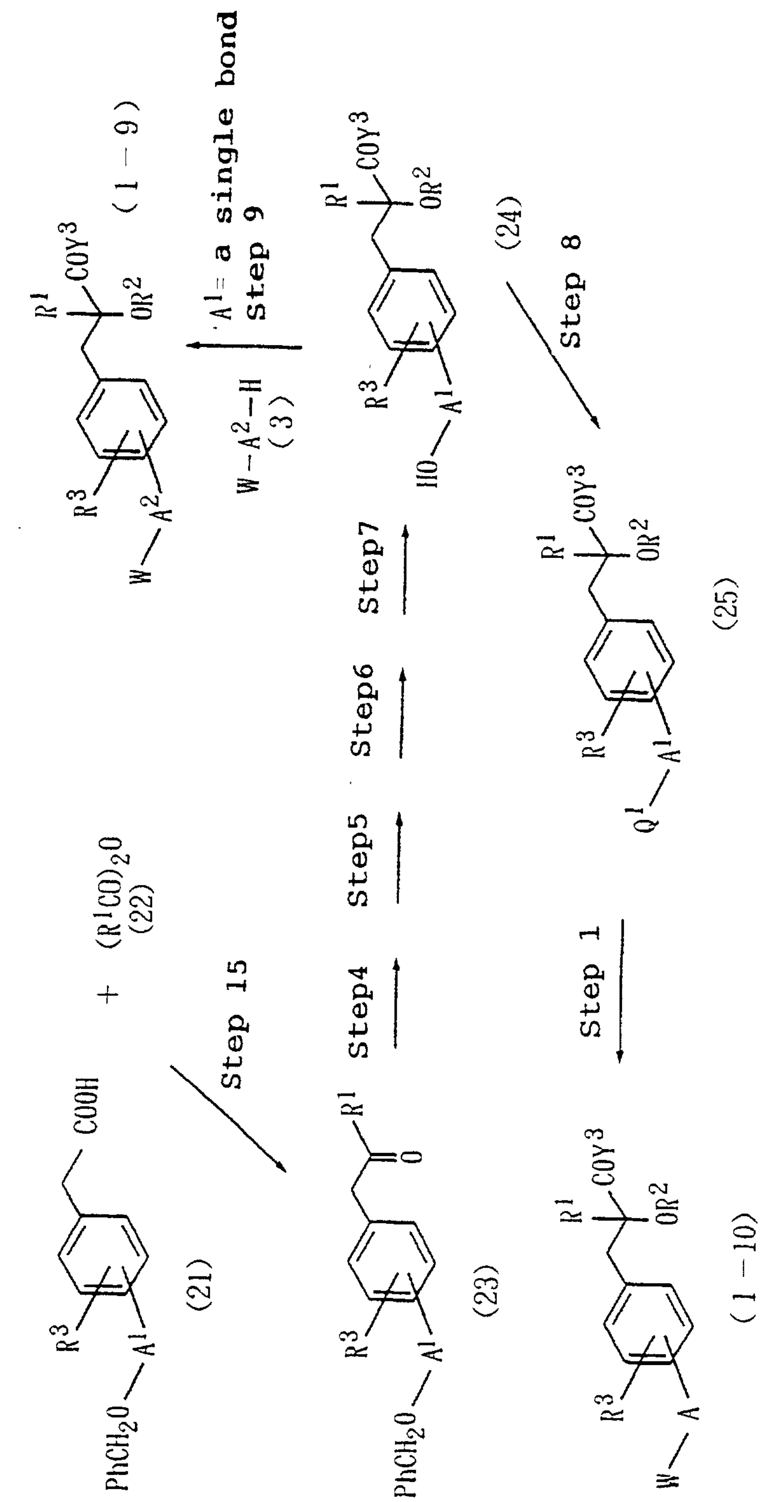


wherein W, A, $A^1$, $A^2$, $Y^3$, $R^1$, $R^2$, $R^3$ and $Q^1$ have the same meanings as defined above.

More specifically, after a compound (21) is reacted with a compound (22) to form a compound (23) (Step 15), exactly the same reactions as in Steps 4, 5, 6 and 7 of Preparation Process 2 are conducted, whereby a compound

(24) can be prepared. After the terminal hydroxy group of the resultant compound (24) is converted into a leaving group ($Q^1$) in the same manner as in Step 8 of Preparation Process 2, the resultant compound is allowed to react in the same manner as in Step 1 of Preparation Process 2, whereby a compound (1-10) according to the present invention can be prepared. When $A^1$ in the compound represented by the general formula (24) is a single bond, the compound is subjected to the Mitsunobu reaction with a compound represented by the general formula (3) in the same manner as in Step 9 of Preparation Process 2, whereby a compound (1-9) according to the present invention can be prepared. Step 15 will hereinafter be described in detail.

Step 15:

The compound (21), which is a starting material, can be purchased as a commercially available reagent or prepared from the compound (9) in accordance with a known method [for example, a method described in Journal of Organic Chemistry, 21, 1149 (1956); Angewandte Chemie, 80, 364 (1968); or the like]. This compound (21) is heated and stirred in the presence of an acid anhydride (22) and a base in accordance with a method described in literature [for example, Journal of American Chemical Society, 72, 1988 (1950); Journal of American Chemical Society, 73, 4911 (1951); Journal of Medicinal Chemistry, 39, 3897 (1996); or the like], whereby the compound (23) can be prepared. Examples of the base used include pyridine, sodium acetate and potassium acetate.

<Preparation Process 7>

Case of $R^1$ = group $COY^2$ in the general formula (1):

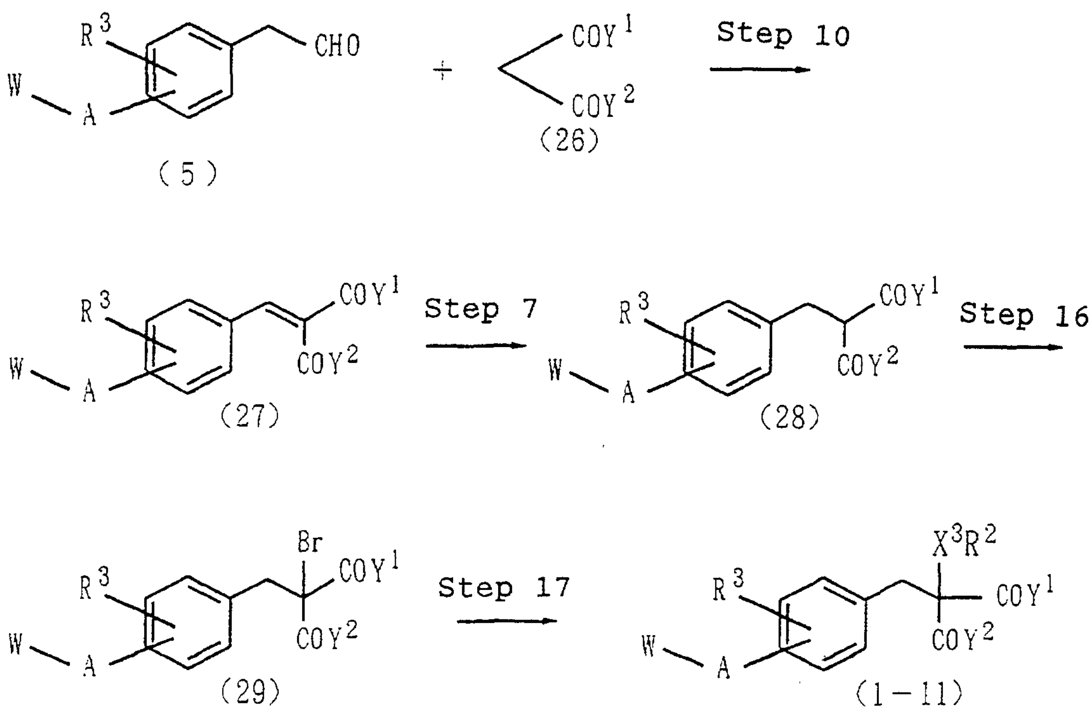


(5) (26) (27) (28) (29) (1-11)

wherein $R^2$, $R^3$, $Y^1$, $Y^2$, A and W have the same meaning as defined above, and $X^3$ is O, S or NH.

More specifically, a compound represented by the general formula (5) is condensed with a malonic acid derivative represented by a general formula (26) in the same manner as in Step 10 of Preparation Process 4, thereby obtaining a compound represented by a general formula (27). The compound thus obtained is reduced in the same manner as in Step 7 of Preparation Process 2 to convert it into a compound represented by a general formula (28), and the compound (28) is then subjected to a brominating reaction, whereby a compound represented by a general formula (29) can be prepared (Step 16). Finally, the compound (29) is reacted with any of various nucleophilic reagents, whereby a compound (1-11) according to the present invention can be prepared (Step 17). Steps 16 and 17 will hereinafter be described in detail.

Step 16:

The compound represented by the general formula (28) is reacted with bromine in the presence of a proper solvent in accordance with a known method [for example, a method described in Organic Synthesis Collect Volume III, 705 (1955); Organic Synthesis Collect Volume I, 245 (1945); Tetrahedron Letters, 24, 163 (1983); or the like], whereby the compound represented by the general formula (29) can be prepared. Examples of the solvent used include acetic acid, diethyl ether, dioxane and carbon tetrachloride.

Step 17:

The compound represented by the general formula (29) is reacted with any of various alcohols, amines and thiols in the presence of proper solvent and base, whereby the compound (1-11) according to the present invention can be prepared. No particular limitation is imposed on the solvent used so far as it does not affect the reaction. Examples thereof include various kinds of alcohols, dioxane, tetrahydrofuran, dimethylformamide and dimethyl sulfoxide. Examples of the base used include sodium hydroxide, potassium hydroxide, potassium carbonate, sodium hydrogen- carbonate, sodium hydride, triethylamine and pyridine. <Preparation Process 8>

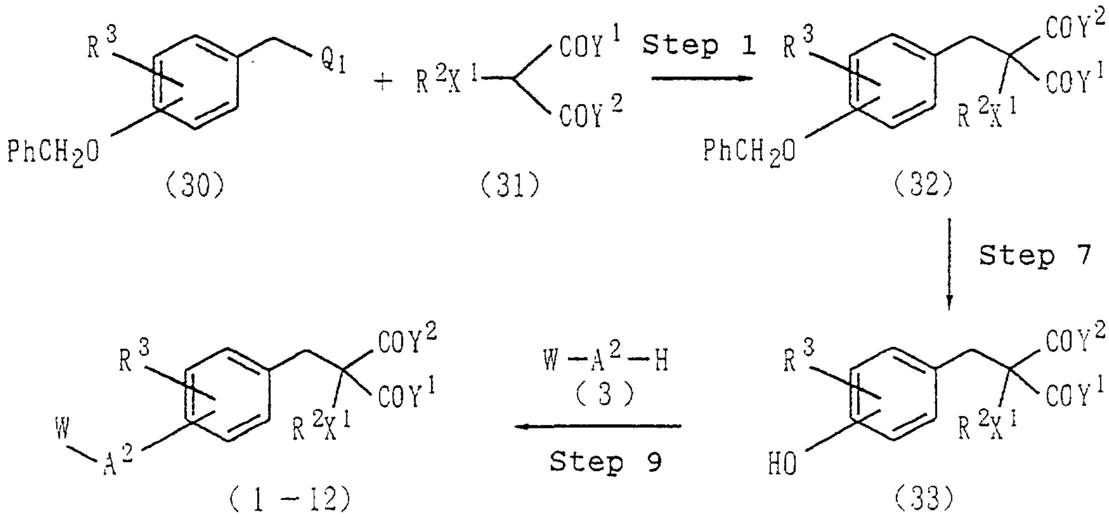


wherein $R^2$, $R^3$, $X^1$, $Y^1$, $Y^2$, $A^2$, $Q^1$ and W have the same meanings as defined above.

More specifically, a compound represented by a general formula (30) is reacted with a compound represented by a general formula (31) in the same manner as in Step 1 of Preparation Process 1, thereby obtaining a compound (32). The compound thus obtained is then catalytically reduced in the same manner as in Step 7 of Preparation Process 2 to obtain a compound (33). Finally, the compound (33) is reacted with the compound (3), whereby a compound (1-12) according to the present invention can be prepared.

The compound (1) according to the present invention obtained by each of the above-described preparation processes can be isolated in the form of crystals or a liquid, as needed, by the conventional means for isolation and purification, for example, recrystallization, distillation and/or chromatography. The compound may also be converted into a salt or solvate as needed.

The compounds (1) according to the present invention are excellent in the effect of lowering blood glucose and lipid and hence useful as medicines for preventing or treating diabetes mellitus, hyperlipemia, obesity and the like.

The medicine according to the present invention is prepared by formulating an effective amount of the compound (1) or the salt thereof as an active ingredient in suitable combination with pharmaceutically acceptable, known carriers, for example, excipients, binders, disintegrators, lubricants, dissolution aids, suspending agents and the like according to pharmacological effects intended, administration object, administration end, administration form, etc. Examples of administration forms include oral administrations by tablets, capsules, granules, powder, syrup, etc., and parenteral administrations by injections, ophthalmic solutions suppositories, etc. In the medicine according to the present invention, the dose of the compound (1) varies according to the condition, age and weight of a patient to be administered, and the administration method thereof. However, the compound (1) may be generally administered in a dose of 0.1 to 1,000 mg per day for an adult.

The compounds (1) according to the present invention may also be used as veterinary medicines for other

mammals than the human.

The present invention will hereinafter be described more specifically by the following Preparation Examples, Examples and Test Example.

Preparation Example 1:

Preparation of 4-[2-(1-oxo-1,2-dihydrophthalazin-2-yl)ethoxy]benzaldehyde (Compound 5):

After dimethylformamide (100 ml) was added to a mixture of 1-phthalazinone (1.46 g), 4-[2-(methane-sulfonyloxy)ethoxy]benzaldehyde (2.44 g) and potassium carbonate (2.07 g), and the mixture was heated and stirred for 4 hours at 85 to 90°C on an oil bath, the reaction mixture was poured into ice water and extracted with ethyl acetate. After the resultant extract was washed with water and then with brine, it was dried over anhydrous sodium sulfate, and the solvent was removed under reduced pressure. The resultant residue was purified by column chromatography on silica gel. A chloroform eluate was crystallized from ether to obtain the title compound (2.17 g, yield: 73.8%) as colorless crystals.

$^1$H-NMR ($CDCl_3$, δ):

4.52(2H,t), 4.68(2H,t), 7.00(2H,d), 7.71-7.86(5H,m), 8.19(1H,s), 8.44(1H,dd), 9.86(1H,s).

Preparation Example 2:

Preparation of 4-[2-(4-oxo-3,4-dihydro-2H-1,3-benzoxazin-3-yl)ethoxy]benzaldehyde (Compound 5):

Dimethyl sulfoxide (100 ml) was added to 2-(4-oxo-3,4-dihydro-2H-1,3-benzoxazin-3-yl)ethanol (11.6 g) and p-fluorobenzaldehyde (11.2 g) to prepare a solution. After sodium hydride (3.6 g; 60% assay) was added portionwise to the solution under ice cooling, the mixture was stirred at room temperature for 2.5 hours. Thereafter, the reaction mixture was poured into ice water, and the precipitate was collected by filtration, washed with water and dried under reduced pressure. After ether was added to the thus-obtained crude crystals, and the resultant mixture was heated and stirred, the mixture was cooled down to room temperature. The precepitate was collected by filtration and dried to obtain the title compound (15.4 g, yield: 86.3%) as colorless crystals.

$^1$H-NMR ($CDCl_3$, δ):

3.99(2H,t), 4.36(2H,t), 5.37(2H,s), 6.97-7.01(3H,m), 7.12(1H,brt), 7.45(1H,ddd), 7.84(2H,d), 7.95(1H,ddd), 9.89 (1H,s).

Preparation Example 3:

Preparation of 2-{2-[4-(2-methoxyvinyl)phenoxy]-ethyl}-1,2-dihydro-1-phthaladinone (Compound 6):

(Methoxymethyl)triphenylphosphonium chloride (13.71 g, 40 mmol) and diisopropylamine (4.22 ml, 30 mmol) were suspended in anhydrous tetrahydrofuran (100 ml). A 1.58 M hexane solution (19 ml, 30 mmol) of n-butyllithium was added to the suspension at -10°C, and the mixture was stirred for 1 hour. A solution of 4-[2-(1-oxo-1,2-dihydrophthalazin-2-yl)ethoxy]benzaldehyde (Compound 5) (5.88 g, 20 mmol) in tetrahydrofuran (40 ml) was then added dropwise to the mixture at the same temperature, and the resultant mixture was stirred for 2 hours at -10°C to room temperature. After completion of the reaction, the reaction mixture was poured into ice water and extracted with ethyl acetate. The resultant extract was dried over anhydrous sodium sulfate, and the solvent was removed under reduced pressure. The resultant residue was purified by column chromatography on silica gel (hexane:ethyl acetate = 4:1) to obtain the title compound (a mixture of E/Z) (6.5 g, yield: 99%) as pale yellow crystals.

$^1$H-NMR ($CDCl_3$, δ): 3.64(1.5H,s), 3.73(1.5H,s), 4.34-4.70(4H,m), 5.10(0.5H,d), 5.75(0.5H,d), 6.05(0.5H,d), 6.80-7.51 (4.5H,m), 7.68-7.84(3H,m), 8.17(1H,s), 8.39-8.48(1H,m).

Preparation Example 4:

Preparation of 2-{2-[4-(2,2-diethoxyethyl)phenoxy]-ethyl}-1,2-dihydro-1-phthaladinone (Compound 7):

Ethanol (200 ml) was added to 2-{2-[4-(2-methoxy-vinyl)phenoxy]ethyl}-1,2-dihydro-1-phthaladinone (Compound 6) (6.44g, 20 mmol) and p-toluenesulfonic acid hydrate (0.38 g, 2 mmol), and the mixture was heated under reflux for 6 hours. After completion of the reaction, the solvent was removed, and ethyl acetate was added to the resultant residue. After the resultant mixture was washed successively with a 5% aqueous solution of sodium hydrogencarbonate and brine and dried over anhydrous magnesium sulfate, the solvent was removed to obtain the title compound (7.2 g, yield: 94.2%) as a pale yellow oil.

$^1$H-NMR ($CDCl_3$, δ): 1.15(6H,t), 2.85(2H,d), 3.34-3.75(4H,m), 4.32-4.72(5H,m), 6.84(2H,d), 7.10(2H,d), 7.69-7.85(3H, m), 8.18(1H,s), 8.34-8.50(1H,m).

Preparation Example 5:

Preparation of 2-ethoxy-3-(4-[2-(1-oxo-1,2-dihydro-phthalazin-2-yl)ethoxy]phenyl}propionitrile (Compound 8):

2-{2-[4-(2,2-Diethoxyethyl)phenoxy]ethyl}-1,2-dihydro-1-phthaladinone (Compound 7) (7.2 g, 18.8 mmol) was dissolved in dichloromethane (100 ml). Trimethylsilyl nitrile (7.52 ml, 56.4 mmol) and boron trifluoride etherate (0.58 ml, 4.7 mmol) were successively added to the solution, and the mixture was stirred for 1 hour. After completion of the reaction, dichloromethane was added to the reaction mixture. After the resultant mixture was washed with a 5% aqueous solution of sodium hydrogencarbonate and water and dried over anhydrous magnesium sulfate, the solvent was removed under reduced pressure. The resultant residue was purified by column chromatography on silica gel (hexane: ethyl acetate = 4:1) to obtain the title compound (6.0 g, yield: 88.0%) as colorless crystals.

$^1$H-NMR ($CDCl_3$, δ):

1.22(3H,t), 3.05(2H,d), 3.30-4.00(2H,m), 4.19(1H,t), 4.40-4.70(4H,m), 6.85(2H,d), 7.15(2H,d), 7.68-7.84(3H,m), 8.17(1H,s), 8.39-8.50(1H,m).

Preparation Example 6:

Preparation of 2-ethoxy-3-[4-(2-hydroxyethyl)-phenyl]propanamide (Compound 11):

2-Ethoxy-3-[4-(2-benzyloxyethyl)phenyl]propanamide (Compound 10) (13.04 g) was dissolved in ethanol (150 ml) and acetic acid (30 ml). To the solution, was added 10% palladium on charcoal (8 g), followed by hydrogenation at room temperature for 4 hours. After completion of the reaction, the reaction mixture was filtered through Celite, and the filtrate was concentrated under reduced pressure. The resultant residue was purified by column chromatography on silica gel ($CHCl_3$:MeOH = 100:2) to obtain the title compound (8.38 g, yield: 88.6%) as a colorless oil.

$^1$H-NMR ($CDCl_3$, δ):

1.14(3H,t), 2.71-4.04(10H,m), 6.18(1H,brs), 6.52(2H,brs), 7.08-7.32(4H,m).

Preparation Example 7:

Preparation of 2-ethoxy-3-{4-[2-(methane-sulfonyloxy)ethyl]phenyl}propanamide (Compound 12):

2-Ethoxy-3-[4-(2-hydroxyethyl)phenyl]propanamide (Compound 11) (8.38 g, 35.3 mmol) and triethylamine (4.29 g, 42.4 mmol) were dissolved in methylene chloride (80 ml). Methanesulfonyl chloride (4.85 g, 42.4 mmol) was added dropwise to the solution under ice cooling, and the resultant mixture was stirred at room temperature for 1 hour. After completion of the reaction, the solvent was removed under reduced pressure, and the residue was dissolved in ethyl acetate. The solution was washed with a saturated aqueous solution of sodium hydrogencarbonate, water and brine in that order, and dried over anhydrous magnesium sulfate. The solvent was then removed under reduced pressure to obtain the title compound (8.44 g, yield: 75.8%) as colorless crystals.

$^1$H-NMR ($CDCl_3$, δ):

1.14(3H,t), 2.78-3.64(9H,m), 3.81-4.05(1H,m), 4.41(2H,t), 5.74(1H,brs), 6.48(1H,brs), 7.08-7.23(4H,m).

Preparation Example 8:

Preparation of 3-[4-(benzyloxy)phenyl]-2-methoxy-2-propenenitrile (Compound 14):

4-Benzyloxybenzaldehyde (2.12 g, 10 mmol) and methoxyacetonitrile (748 mg, 10 mmol) were dissolved in dimethylformamide (30 ml). Sodium hydride (60% assay; 480 mg, 12 mmol) was added to the solution at room temperature, and the resultant mixture was heated and stirred for 1 hour at 110°C on an oil bath. After completion of the reaction, the reaction mixture was poured into ice water and extracted with ethyl acetate. After the resultant extract was washed with brine and dried over anhydrous magnesium sulfate, the solvent was removed under reduced pressure. The resultant residue was purified by column chromatography on silica gel (hexane:ethyl acetate = 10:1) to obtain the title compound (a mixture of E/Z; 1.58 g, yield: 59.7%) as colorless syrup.

$^1$H-NMR ($CDCl_3$, δ):

3.76(1.5H,s), 3.90(1.5H,s), 5.09(2H,s), 6.14(0.5H,s), 6.53(0.5H,s), 6.95(1H,d), 6.98(1H,d), 7.10-7.40(5H,m), 7.51(1H,d), 7.58(1H,d).

Example 1:

Preparation of 2-ethoxy-3-{4-[2-(1-oxo-1,2-dihydro-phthalazin-2-yl)ethoxy]phenyl}propanamide [Invention Compound (1A)]:

2-Ethoxy-3-{4-[2-(1-oxo-1,2-dihydrophthalazin-2-yl)ethoxy]phenyl}propionitrile (Compound 8) (6.0 g, 16.5

mmol) was dissolved in ethanol (200 ml), and a 6N aqueous solution (8.25 ml, 49.5 mmol) of sodium hydroxide was added to the solution. The resultant mixture was heated under reflux for 1.5 hours. After completion of the reaction, water was added to the reaction mixture to conduct extraction with ethyl acetate. The resultant extract was washed with brine and dried over anhydrous magnesium sulfate. The solvent was then removed under reduced pressure. The resultant residue was purified by column chromatography on silica gel (hexane:ethyl acetate = 4:1) and recrystallized from ethyl acetate to obtain Invention Compound (1A) (3.3 g, yield: 52.5%) as colorless crystals.

Example 2:

Invention Compounds (1D), (1F), (1H), (1J), (1K), (1N), (1AB), (1AC), (1BG), (1BU) and (1BV) were prepared in the same manner as in Example 1.

Example 3:

Preparation of 2-ethoxy-3-{4-[2-(4-oxo-3,4-dihydro-quinazolin-3-yl)ethyl]phenyl}propanamide [Invention Compound (1Q)]:

4-Quinazoline (500 mg, 3.42 mmol), 2-ethoxy-3-{4-[2-(methanesulfonyloxy)ethyl]phenyl)propanamide (Compound 12) (1.13 g, 3.42 mmol) and potassium carbonate (1.42 g, 10.3 mmol) were dissolved in dimethylformamide (20 ml), and the resultant solution was heated and stirred at 80°C for 2 hours. After cooling the reaction mixture, water was added to the reaction mixture to conduct extraction with ethyl acetate. The resultant extract was washed with water and then dried over anhydrous magnesium sulfate. The solvent was removed under reduced pressure. The precipitate was collected by filtration and washed with ether. The crude crystals thus obtained were recrystallized from ethanol to obtain Invention Compound (1Q) (1.11 g, yield: 87.6%).

Example 4:

Invention Compounds (1E), (1G), (1I), (1S), (1T), (1U), (1V), (1W), (1AF), (1AI) and (1BR) were prepared in the same manner as in Example 3.

Example 5:

Preparation of 2-methoxy-3-{4-[2-(4-oxo-3,4-dihydro-2H-1,3-benzoxazin-3-yl)ethoxy]phenyl}propanamide [Invention Compound (1P)]:

2-(4-Oxo-3,4-dihydro-2H-1,3-benzoxazin-3-yl)ethanol (794 mg, 4.11 mmol), 2-methoxy-3-(4-hydroxyphenylpropanamide (802 mg, 4.11 mmol) and triphenylphosphine (1.13 g, 4.31 mmol) were dissolved in tetrahydrofuran (20 ml), and a 40% toluene solution of diethyl azodicarboxylate (1.88 g, 4.31 mmol) was added dropwise to the solution under ice cooling. The resultant mixture was stirred overnight at room temperature. After completion of the reaction, the solvent was removed under reduced pressure. Ether was added to the residue, and the resultant mixture was washed with a 10% aqueous solution of sodium hydroxide and brine. An organic layer was dried over anhydrous magnesium sulfate. The solvent was then removed under reduced pressure, and the resultant residue was purified by column chromatography on silica gel (hexane:ethyl acetate = 4:1) to obtain Invention Compound (1P) (976 mg, yield: 64.1%) as colorless crystals.

Example 6:

Invention Compounds (1R), (1AH), (1AJ), (1AK), (1AL), (1AM), (1AS), (1AT), (1AV), (1AY), (1AZ) and (1BE) were prepared in the same manner as in Example 5.

Example 7:

Preparation of ethyl 2-ethylamino-3-{4-[2-(4-oxo-3,4-dihydro-2H-1,3-benzoxazin-3-yl)ethoxy]phenyl}-propionate [Invention Compound (1L)]:

Ethyl 2-amino-3-{4-[2-(4-oxo-3,4-dihydro-2H-1,3-benzoxazin-3-yl)ethoxy]phenyl}propionate hydrochloride (1.05 g, 2.5 mmol) was dissolved in ethanol (10 ml), and ethyl iodide (390 mg, 2.5 mmol) and diisopropylethylamine (323 mg, 2.5 mmol) were added dropwise to the solution. The resultant mixture was stirred for 2 days at 60°C [during which additional ethyl iodide and diisopropylethylamine (each, 5 mmol) were added]. After completion of the reaction, the solvent was removed, and ethyl acetate was added to the residue. The resultant mixture was washed with water and dried over anhydrous magnesium sulfate. The solvent was then removed under reduced pressure, and the resultant

residue was purified by column chromatography on silica gel to obtain Invention Compound (1L) (627 mg, yield: 60.8%) as a colorless oil.

Example 8:

Preparation of 2-ethylamino-3-{4-[2-(4-oxo-3,4-dihydro-2H-1,3-benzoxazin-3-yl)ethoxy]phenyl}propionic acid [Invention Compound (1M)]:

A 1N aqueous solution (5 ml) of sodium hydroxide was added to a solution of ethyl 2-ethylamino-3-{4-[2-(4-oxo-3,4-dihydro-2H-1,3-benzoxazin-3-yl)ethoxy]phenyl}-propionate (627 mg) in ethanol (8 ml), and the resultant mixture was stirred overnight at 60°C. After the completion of a reaction was confirmed by using TLC, the reaction mixture was concentrated under reduced pressure. Water was added to the residue, and the resultant mixture was neutralized with 3% hydrochloric acid. The precipitate was then collected by filtration, washed with water and then dried to obtain Invention Compound (1M) (640 mg, quantitative) in the form of the hydrochloride as colorless crystals.

Example 9:

Invention Compounds (1BA), (1BB) and (1BF) were prepared in the same manner as in Example 8.

Example 10:

Preparation of 2-ethylthio-3-{4-[2-(4-oxo-3,4-dihydro-2H-1,3-benzoxazin-3-yl)ethoxy]phenyl}propanoic acid [Invention Compound (10)]:

A 15% aqueous solution (60 ml) of sodium hydroxide was added to 5-{4-[2-(4-oxo-3,4-dihydro-2H- 1,3-benzoxazin-3-yl)ethoxy]benzyl}-2,4-thiazolidinedione (3.0 g). After the resultant mixture was gently heated to reflux for 30 minutes, methanol (60 ml) was then added to the mixture at room temperature, and a methanol solution of ethyl iodide (5.3 g) was then added dropwise, followed by stirring for 1.5 hours. Thereafter, the reaction mixture was poured into ice water, acidified with hydrochloric acid and extracted with ethyl acetate. The resultant extract was washed with brine and dried over anhydrous sodium sulfate. The solvent was then removed under reduced pressure. The resultant residue was purified by column chromatography on silica gel (chloroform:methanol = 50:1) and crystallized from hexane-ethyl acetate to obtain Invention Compound (10) (600 mg, yield: 19.9%) as colorless crystals.

Example 11:

Preparation of 2-ethoxy-3-{4-[2-(1-oxo-1,2-dihydro-phthalazin-2-yl)ethoxy]phenyl}propanoic acid [Invention Compound (1B)]:

2-Ethoxy-3-{4-[2-(1-oxo-1,2-dihydrophthalazin-2-yl)ethoxy]phenyl}propanamide [Invention Compound (1A)] (12.55 g, 32.9 mmol) was dissolved in dioxane (148 ml), and 2N hydrochloric acid (16.5 ml, 32.9 mmol) and a catalytic amount of $TiCl_4$ were added to the solution, followed by stirring at 110°C for 6 hours. After completion of the reaction, the solvent was removed, and chloroform was added to the residue. The precipitate was removed by filtration, and the filtrate was dried over anhydrous magnesium sulfate. The solvent was then removed, and the resultant residue was purified by column chromatography on silica gel (hexane:ethyl acetate = 2:3) and recrystallized from ethyl acetate to obtain Invention Compound (1B) (5.23 g, yield: 41.8%) as colorless crystals.

Example 12:

Invention Compounds (1AE), (1BC), (1BD), (1BJ), (1BK), (1BL) and (1BS) were prepared in the same manner as in Example 11.

Example 13:

Preparation of ethyl 2-ethoxy-3-{4-[2-(1-oxo-1,2-dihydrophthalazin-2-yl)ethoxy]phenyl}propanoate [Invention Compound (1C)]:

2-Ethoxy-3-{4-[2-(1-oxo-1,2-dihydrophthalazin-2-yl)ethoxy]phenyl}propanamide [Invention Compound (1A)] (1.0 g, 2.62 mmol) was dissolved in ethanol (30 ml), and 1N hydrochloric acid (2.6 ml, 2.6 mmol) and a catalytic amount of $TiCl_4$ were added to the solution, followed by refluxing for 5 hours. After completion of the reaction, the solvent was removed, and chloroform was added to the residue. The precipitate was removed by filtration, and the filtrate was dried over anhydrous magnesium sulfate. The resultant residue was then purified by column chromatography on silica gel (hexane:ethyl acetate = 2:3) to obtain Invention Compound (1C) (0.2 g, yield: 20.0%) as a colorless oil.

Example 14:

Invention Compounds (1AN), (1AO), (1AP), (1AQ), (1AU), (1AW), (1AX) and (1BO) were prepared in the same manner as in Example 13.

Example 15:

Preparation of 2-ethoxy-3-{4-[2-(1-oxo-1,2-dihydro-phthalazin-2-yl)ethoxy]phenyl}propanohydroxamic acid [Invention Compound (1AA)]:

2-Ethoxy-3-{4-[2-(1-oxo-1,2-dihydrophthalazin-2-yl)ethoxy]phenyl}propanoic acid [Invention Compound (1B)] (500 mg, 1.31 mmol) was dissolved in dimethylformamide (10 ml), and carbonyldiimidazole (233 mg, 1.44 mmol) was added to the solution. After the resultant mixture was stirred at room temperature for 1 hour, hydroxyamine hydrochloride (200 mg, 2.88 mmol) was added, and the mixture was stirred at the same temperature for 12 hours. After completion of the reaction, water was added to the reaction mixture to conduct extraction with ethyl acetate. The resultant extract was washed with water and then with brine, and dried over anhydrous magnesium sulfate. The solvent was then removed, and the residue was purified by column chromatography on silica gel (ethyl acetate:hexane = 3:2 to 4:1) and recrystallized from a mixed solvent of ethyl acetate and ether to obtain Invention Compound (1AA) (150 mg, yield: 28.8%) as colorless crystals.

Example 16:

Invention Compounds (1Z), (1AD), (1AG), (1BH) and (1BP) were prepared in the same manner as in Example 15. Example 17:

Preparation of ethyl 2-ethoxy-3-{4-[2-(4-methyl-1-oxo-1,2-dihydrophthalazin-2-yl)ethoxy]phenyl}propanoate [Invention Compound (1BI)]:

Ethyl 2-ethoxy-3-{4-[2-(4-methyl-1-oxo-1,2-dihydro-phthalazin-2-yl)ethoxy]phenyl}propenoate (4.96 g, 11.75 mmol) was dissolved in ethyl acetate (60 ml), and 10% palladium on carbon (600 mg) was added to conduct catalytic reduction for 10 hours. After completion of the reaction, the catalyst was removed by filtration, and the filtrate was concentrated. The resultant residue was purified by column chromatography on silica gel to obtain Invention Compound (1BI) (4.54 g, yield: 91.2%) as colorless crystals.

Example 18:

Invention Compounds (1AR), (1BM), (1BN), (1BQ), (1BT) and (1BW) were prepared in the same manner as in

Example 17.

The structures and physical data of the compounds obtained in the above-described Examples are shown in the following tables. W, A, $R^1$, $R^2$, $R^3$, X and $Y^1$ in the tables mean the respective symbols in the general formula (1). The position of substitution (abbreviated as "PS" in the tables) indicates the position of W-A- substituted on a benzene ring.

Table 1

| No. | W | A | PS | $R^1$ | $R^2$ | $R^3$ | X | $Y^1$ | m.p. (℃) | NMR ($CDCl_3$, δ) |
|---|---|---|---|---|---|---|---|---|---|---|
| 1A | 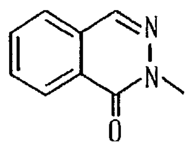 | $-CH_2-CH_2-O-$ | 4 | H | Et | H | O | $NH_2$ | 128-129 | 1.11(3H, t), 2.85(1H, dd), 3.05(1H, dd), 3.38-3.52(2H, m), 3.85(1H, dd), 4.40(2H, t), 4.64(2H, t), 5.48(1H, br), 6.42(1H, br), 6.83(2H, d), 7.12(2H, d), 7.69-7.84(3H, m), 8.19(1H, s), 8.45(1H, dd) |
| 1B | 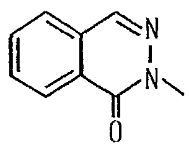 | $-CH_2-CH_2-O-$ | 4 | H | Et | H | O | OH | 113-115 | 1.15(3H, t), 2.91(1H, dd), 3.05(1H, dd), 3.38-3.61(2H, m), 4.02(1H, dd), 4.40(2H, t), 4.64(2H, t), 6.85(2H, d), 7.13(2H, d), 7.69-7.84(3H, m), 8.19(1H, s), 8.43-8.45(1H, m) |
| 1C | 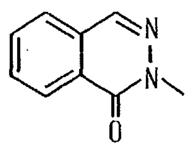 | $-CH_2-CH_2-O-$ | 4 | H | Et | H | O | OEt | oil | 1.14(3H, t), 1.21(3H, t), 2.91-2.93(2H, m), 3.29-3.36(1H, m), 3.54-3.62(1H, m), 3.93(1H, dd), 4.14-4.18(2H, m), 4.40(2H, t), 4.64(2H, t), 6.84(2H, d), 7.12(2H, d), 7.69-7.84(3H, m), 8.18(1H, s), 8.43-8.45(1H, m) |
| 1D | 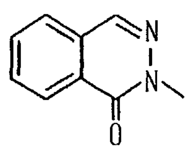 | $-CH_2-CH_2-O-$ | 4 | H | Et | 3-OMe | O | $NH_2$ | 137-140 | 1.12(3H, t), 2.82(1H, dd), 3.07(1H, dd), 3.37-3.54(2H, m), 3.77(3H, s), 3.87(1H, dd), 4.45(2H, t), 4.67(2H, t), 5.50(1H, br), 6.45(1H, br), 6.74-6.78 (2H, m), 6.90(1H, d), 7.69-7.84(3H, m), 8.19(1H, s), 8.43-8.45(1H, m) |

Table 2

| No. | W | A | PS | $R^1$ | $R^2$ | $R^3$ | X | $Y^1$ | m.p. (℃) | NMR($CDCl_3$, δ) |
|---|---|---|---|---|---|---|---|---|---|---|
| 1E | 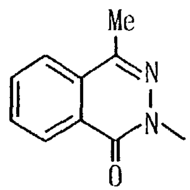 | $-CH_2-CH_2-O-$ | 4 | H | Et | H | O | $NH_2$ | 141-145 | 1.12(3H, t), 2.60(3H, s), 2.85(1H, dd), 3.06(1H, dd), 3.36-3.53(2H, m), 3.86(1H, dd), 4.39(2H, t), 4.60(2H, t), 5.42(1H, br), 6.42(1H, br), 6.84(2H, d), 7.12(2H, d), 7.74-7.84(3H, m), 8.46-8.48(1H, m) |
| 1F | 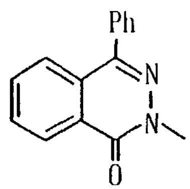 | $-CH_2-CH_2-O-$ | 4 | H | Et | H | O | $NH_2$ | 87-88 | 1.11(3H, t), 2.83(1H, dd), 3.05(1H, dd), 3.32-3.52(2H, m), 3.85(1H, dd), 4.44(2H, t), 4.71(2H, t), 5.59(1H, brs), 6.42(1H, brs), 6.85(2H, d), 7.12(2H, d), 7.52-7.59(5H, m), 7.72-7.81(3H, m), 8.53-8.55(1H, m) |
| 1G | 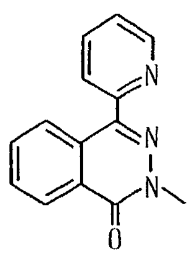 | $-CH_2-CH_2-O-$ | 4 | H | Et | H | O | $NH_2$ | 138-140 | 1.11(3H, t), 2.83(1H, dd), 3.05(1H, dd), 3.35-3.52(2H, m), 3.85(1H, dd), 4.45(2H, t), 4.73(2H, t), 5.51(1H, br), 6.41(1H, br), 6.84(2H, d), 7.12(2H, d), 7.40-7.43(1H, m), 7.76-7.90(4H, m), 8.42-8.45(1H, m), 8.50-8.53(1H, m), 8.75-8.77(1H, m) |
| 1H | 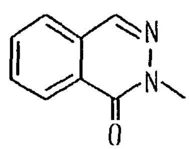 | $-CH_2-CH(OH)-CH_2-O-$ | 4 | H | Et | H | O | $NH_2$ | 104-106 | 1.14(3H, t), 2.86(1H, dd), 3.08(1H, dd), 3.40-3.55(2H, m), 3.80(1H, d), 3.89(1H, dd), 4.05-4.13(2H, m), 4.47-4.62(3H, m), 5.40(1H, br), 6.43(1H, br), 6.85(2H, d), 7.15(2H, d), 7.72-7.74(1H, m), 7.79-7.87(2H, m), 8.21(1H, s), 8.44(1H, d) |

Table 3

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1I | 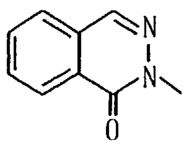 | $-CH_2-CH_2-$ | 4 | H | Et | H | O | $NH_2$ | 146-149 | 1.13(3H, t), 2.86(1H, dd), 3.09-3.15(3H, m), 3.40-3.53(2H, m), 3.90(1H, dd), 4.43-4.47(2H, m), 5.53(1H, br), 6.44(1H, br), 7.18(2H, d), 7.20(2H, d), 7.68-7.83(3H, m), 8.16(1H, s), 8.42(1H, dd) |
| 1J | 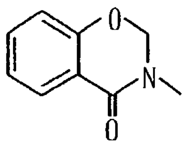 | $-CH_2-CH_2-O-$ | 4 | H | Et | H | O | $NH_2$ | 101-102 | 1.13(3H, t), 2.86(1H, dd), 3.07(1H, dd), 3.38-3.54(2H, m), 3.87(1H, dd), 3.95(2H, t), 4.18(2H, t), 5.3 (1H, brs), 5.37(2H, s), 6.40(1H, brs), 6.79(2H, d), 6.97(1H, dd), 7.09-7.14(1H, m), 7.15(2H, d), 7.42-7.46(1H, m), 7.95(1H, dd) |
| 1K | 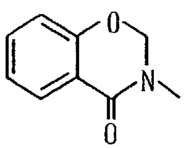 | $-CH_2-CH_2-O-$ | 4 | H | Et | H | O | OH | 98-99 | 1.16(3H, t), 2.94(1H, dd), 3.06(1H, dd), 3.38-3.46(1H, m), 3.55-3.63(1H, m), 3.95(2H, t), 4.02(1H, dd), 4.17(2H, t), 5.37(2H, s), 6.80(2H, d), 6.97(1H, d), 7.10(1H, dt), 7.42-7.46(1H, m), 7.95(1H, dd) |
| 1L | 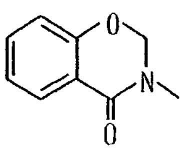 | $-CH_2-CH_2-O-$ | 4 | H | Et | H | NH | OEt | Oil | 1.06(3H, t), 1.15(3H, t), 2.48-2.58(1H, m), 2.60-2.67(1H, m), 2.82-2.93(2H, m), 3.46(1H, t), 3.94(2H, t), 4.09(2H, q), 4.17(2H, t), 5.36(2H, s), 6.79(2H, d), 6.96(1H, d), 7.07-7.12(3H, m), 7.43(1H, dt), 7.95(1H, dd) |

Table 4

| No | W | A | PS | $R^1$ | $R^2$ | $R^3$ | X | $Y^1$ | m. p. (°C) | NMR ($CDCl_3$, δ) |
|---|---|---|---|---|---|---|---|---|---|---|
| 1M | 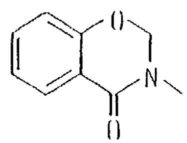 | $-CH_2-CH_2-O-$ | 4 | H | Et | H | NH | OH | 228 229 (HCl salt) | 0. 99(3H, t), 2. 38 2. 49(1H, m), 2. 50 2. 55(1H, m), 2. 72 2. 83(2H, m), 3. 26(1H, t), 3. 72(2H, t), 4. 03(2H, t), 4. 81(2H, s), 6. 74 6. 77(3H, m), 6. 97(1H, t), 7. 09(2H, d), 7. 30(1H, dt), 7. 66(1H, dd), ($D_2O$+NaOD)(HCl salt) |
| 1N | 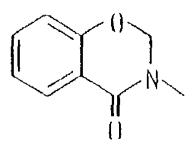 | $-CH_2-CH_2-O-$ | 1 | H | $CH_3$<br>\|<br>—CH—<br>\|<br>$CH_3$ | H | O | $NH_2$ | 82 83 | 0. 95(3H, d), 1. 11(3H, d), 2. 80(1H, dd), 3. 05(1H, dd), 3. 45(1H, ddd), 3. 92(1H, dd), 3. 95(2H, t), 4. 18(2H, t), 5. 37(2H, s), 5. 43(1H, br), 6. 49(1H, br), 6. 79(2H, d), 6. 97(1H, d), **7.11 (1H, dt)**, 7. 16(2H, d), 7. 42 7. 46(1H, m), 7. 95(1H, dd) |
| 1O | 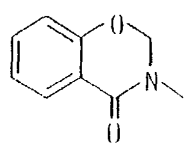 | $-CH_2-CH_2-O-$ | 4 | H | Et | H | S | OH | 71 72 | 1. 22(3H, t), 2. 61 2. 68(2H, m), 2. 89(1H, dd), 3. 13(1H, dd), 3. 47(1H, dd), 3. 94(2H, t), 4. 14(2H, t), 5. 35(2H, s), 6. 79(2H, d), 6. 96(1H, d), 7. 08 7. 14(3H, m), 7. 41 7. 45(1H, m), 7. 93(1H, dd) |
| 1P | 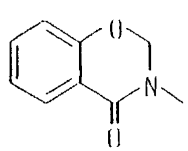 | $-CH_2-CH_2-O-$ | 4 | H | Me | H | O | $NH_2$ | 142 143 | 2. 89(1H, dd), 3. 08(1H, dd), 3. 31(3H, s), 3. 81(1H, dd), 3. 95(2H, t), 4. 18(2H, t), 5. 32(1H, br d), 5. 37(2H, s), 6. 32(1H, br d), 6. 80(2H, d), 6. 97(1H, dd), 7. 11(1H, dt), 7. 15(2H, d), 7. 42 7. 46(1H, m), 7. 95(1H, dd) |

Table 5

| No. | W | A | PS | $R^1$ | $R^2$ | $R^3$ | X | $Y^1$ | m.p. (°C) | NMR ($CDCl_3$, δ) |
|---|---|---|---|---|---|---|---|---|---|---|
| 1Q | 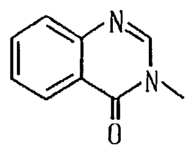 | $-CH_2-CH_2-O-$ | 4 | H | Et | H | O | $NH_2$ | 156-157 | 1.02(3H, t), 2.70(1H, dd), 2.84(1H, dd), 3.22-3.30(1H, m), 3.39-3.47(1H, m), 3.72(1H, d), 4.26(2H, t), 4.37(2H, t), 6.81-6.85(2H, m), 7.04-7.11(4H, m), 7.55(1H, ddd), 7.69(1H, dd), 7.83(1H, ddd), 8.17(1H, dd), 8.38(1H, s)(DMSO-$d_6$) |
| 1R | 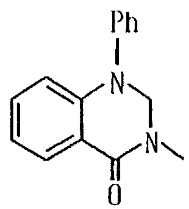 | $-CH_2-CH_2-O-$ | 4 | H | Et | H | O | $NH_2$ | amorphous | 1.12(3H, t), 2.84(1H, dd), 3.06(1H, dd), 3.36-3.44(1H, m), 3.46-3.53(1H, m), 3.86(1H, dd), 3.90(2H, t), 4.15(2H, t), 5.18(2H, s), 5.45(1H, brs), 6.42(1H, brs), 6.61-6.64(2H, m), 6.90(1H, dd), 7.00(1H, ddd), 7.09-7.11(2H, m), 7.14-7.18(3H, m), 7.29(1H, ddd), 7.32(2H, ddd), 8.02(1H, dd) |
| 1S | 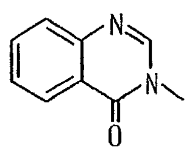 | $-CH_2-CH_2-$ | 4 | H | Et | H | O | $NH_2$ | 144-145 | 1.01(3H, t), 2.74(1H, dd), 2.91(1H, dd), 2.99(2H, t), 3.21-3.29(1H, m), 3.40-3.47 (1H, m), 3.77(1H, dd), 4.20(2H, t), 7.07-7.16(6H, m), 7.54(1H, ddd), 7.63(1H, dd), 7.81(1H, ddd), 8.13(1H, s), 8.18(1H, dd) (DMSO-$d_6$) |
| 1T | 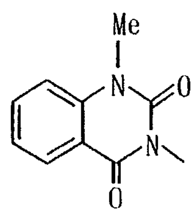 | $-CH_2-CH_2-O-$ | 4 | H | Et | H | O | $NH_2$ | 134-135 | 1.03(3H, t), 2.71(1H, dd), 2.85(1H, dd), 3.21-3.31(1H, m), 3.41-3.48(1H, m), 3.53(3H, s), 3.74(1H, dd), 4.16(2H, t), 4.33(2H, t), 6.81-6.85(2H, m), 7.05-7.11(4H, m), 7.31(1H, dd), 7.78(1H, ddd), 8.07(1H, dd)(DMSO-$d_6$) |

Table 6

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1U | 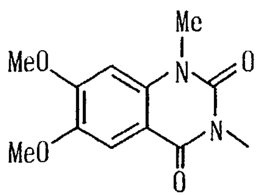 | $-CH_2-CH_2-O-$ | 4 | H | Et | H | O | $NH_2$ | 183-184 | 1.03(3H, t), 2.71(1H, dd), 2.85(1H, dd), 3.22-3.32(1H, m), 3.41-3.48(1H, m), 3.55(3H, s), 3.74(1H, dd), 3.83(3H, s), 3.95(3H, s), 4.15(2H, t), 4.31(2H, t), 6.82-6.85(2H, m), 6.89(1H, s), 7.05-7.11(4H, m), 7.44(1H, s) (DMSO-$d_6$) |
| 1V | 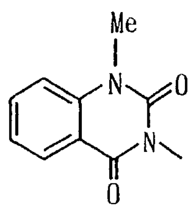 | $-CH_2-CH_2-$ | 4 | H | Et | H | O | $NH_2$ | 187-188 | 1.03(3H, t), 2.76(1H, dd), 2.83-2.87(2H, m), 2.91(1H, dd), 3.23-3.31(1H, m), 3.41-3.49(1H, m), 3.53(3H, s) 3.78(1H, dd), 4.12-4.16(2H, m), 7.08-7.19(6H, m), 7.45(1H, dd), 7.77(1H, ddd), 8.05(1H, dd) (DMSO-$d_6$) |
| 1W | 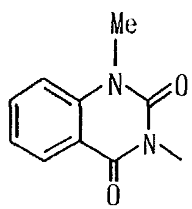 | $-CH_2-CH_2-CH_2-$ | 4 | H | Et | H | O | $NH_2$ | 112-113 | 1.03(3H, t), 1.90(2H, quin), 2.61(2H, t), 2.73(1H, dd), 2.87(1H, dd), 3.25-3.50(5H, m), 3.77(1H, dd), 3.99(2H, t), 7.06-7.14(6H, m), 7.29(1H, ddd), 7.42(1H, dd), 7.76(1H, dd), 8.04(1H, dd) (DMSO-$d_6$) |

Table 7

| No. | W | A | PS | $R^1$ | $R^2$ | $R^3$ | X | $Y^1$ | m.p. (°C) | NMR ($CDCl_3$, δ) |
|---|---|---|---|---|---|---|---|---|---|---|
| 17 | 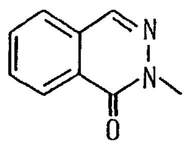 | $-CH_2-CH_2-O-$ | 4 | H | Et | H | O | NHMe | 103-104 | 1.10(3H, t), 2.75(3H, d), 2.81(1H, dd), 3.06(1H, dd), 3.33-3.48(2H, m), 4.12(1H, dd), 4.40(2H, t), 4.64(2H, t), 6.44(1H, m), 6.83(2H, d), 7.08(2H, d), 7.69-7.84(3H, m), 8.18(1H, s), 8.43-8.45(1H, m) |

Table 8

| No. | W | A | PS | $R^1$ | $R^2$ | $R^3$ | X | $Y^1$ | m. p. (℃) | NMR ($CDCl_3$, δ) |
|---|---|---|---|---|---|---|---|---|---|---|
| 1AA | 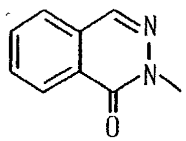 | $-CH_2-CH_2-O-$ | 4 | H | Et | H | O | NHOH | 145-148 | 1.09(3H, t), 2.83(1H, dd), 3.07(1H, dd), 3.34-3.49(2H, m), 4.00(1H, dd), 4.40(2H, t), 4.63(2H, t), 6.83(2H, d), 7.08(2H, d), 7.69-7.84(4H, m), 8.19(1H, s), 8.42-8.44(1H, m), 8.85(1H, s) |
| 1AB | 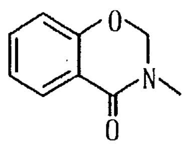 | $-CH_2-CH_2-O-$ | 4 | H | Et | 3-OMe | O | $NH_2$ | 160-161 | 1.13(3H, t), 2.84(1H, dd), 3.07(1H, dd), 3.39-3.53(2H, m), 3.80(3H, s), 3.87(1H, dd), 3.97(2H, t), 4.20(2H, t), 5.43(2H, s), 5.51(1H, br s), 6.45(1H, br s), 6.74-6.80(3H, m), 6.97(1H, dd), 7.10(1H, dt), 7.41-7.46(1H, m), 7.95(1H, dd) |
| 1AC | 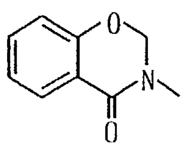 | $-CH_2-CH_2-O-$ | 4 | H | Et | 3-OMe | O | OH | 82-83 (Na salt) | 0.98(3H, t), 2.60(1H, dd), 2.84(1H, dd), 3.09-3.18(1H, m), 3.52-3.61(1H, m), 3.70(3H, s), 3.84(2H, t), 4.08(2H, t), 5.42(2H, s), 6.69(1H, dd), 6.82-6.86(2H, m), 7.05(1H, d), 7.13-7.16(1H, m), 7.52(1H, dt), 7.81(1H, dd), (Na salt DMSO-$d_6$) |
| 1AD | 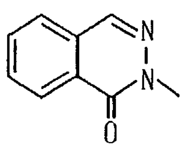 | $-CH_2-CH_2-O-$ | 4 | H | Et | H | O | 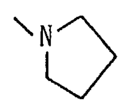 | oil | 1.17(3H, t), 1.66-1.76(4H, m), 2.94-2.99(3H, m), 3.31-3.54(5H, m), 4.11(1H, t), 4.39(2H, t), 4.64(2H, t), 6.83(2H, d), 7.12(2H, d), 7.69-7.84(3H, m), 8.18(1H, s), 8.43-8.45(1H, m) |

Table 9

| No. | W | A | PS | $R^1$ | $R^2$ | $R^3$ | X | $Y^1$ | m.p. (℃) | NMR ($CDCl_3$, δ) |
|---|---|---|---|---|---|---|---|---|---|---|
| 1AE | 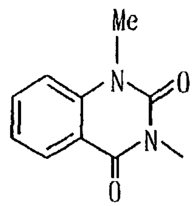 | $-CH_2-CH_2-O-$ | 4 | H | Et | H | O | OH | 89-90 | 1.16(3H, t), 2.91(1H, dd), 3.05(1H, dd), 3.38-3.46(1H, m), 3.55-3.63(4H, m), 4.02(1H, dd), 4.25(2H, t), 4.53(2H, t), 7.10-7.14(2H, m), 7.20-7.29(2H, m), 7.69(1H, ddd), 8.24(1H, dd) |
| 1AF | 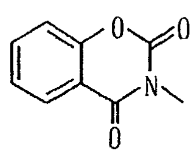 | $-CH_2-CH_2-O-$ | 4 | H | Et | H | O | $NH_2$ | 172-173 | 1.03(3H, t), 2.72(1H, dd), 2.86(1H, dd), 3.24-3.31(1H, m), 3.41-3.49(1H, m), 3.74(1H, dd), 4.20(2H, t), 4.26(2H, t), 6.82(2H, d), 7.06(1H, br s), 7.10-7.12(3H, m), 7.41-7.46(2H, m), 7.80-7.84(1H, m), 8.00(1H, dd) ($DMSO-d_6$) |
| 1AG | 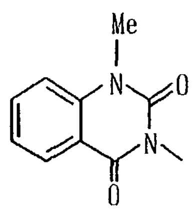 | $-CH_2-CH_2-O-$ | 4 | H | Et | H | O | NHOH | 146-148 | 1.09(3H, t), 2.82(1H, dd), 3.07(1H, dd), 3.34-3.38(1H, m), 3.43-3.49(1H, m), 3.61(3H, s), 3.98(1H, dd), 4.25(2H, t), 4.53(2H, t), 6.82-6.85(2H, m), 7.08-7.10(2H, m), 7.18-7.29(2H, m), 7.69(1H, ddd), 8.23(1H, dd), 8.34(1H, br), 8.90(1H, br s) |

Table 10

| No. | W | A | PS | $R^1$ | $R^2$ | $R^3$ | X | $Y^1$ | m. p. (℃) | NMR ($CDC\ell_3$, δ) |
|---|---|---|---|---|---|---|---|---|---|---|
| 1AH | 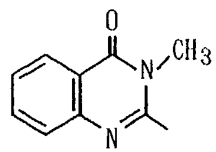 | $-CH_2-CH_2-O-$ | 4 | H | $CH_2CH_3$ | H | O | $NH_2$ | 155-156.5 | 1.05(3H, t), 2.74(1H, dd), 2.88(1H, dd), 3.21-3.37(3H, m), 3.42-3.50(1H, m), 3.59 (3H, s), 3.76(1H, dd), 4.47(2H, t), 6.87-6.91(2H, m), 7.06(1H, brs), 7.13-7.15 (3H, m), 7.48(1H, ddd), 7.59(1H, dd), 7.78(1H, ddd), 8.12(1H, dd) ($DMSO-d_6$) |
| 1AI | 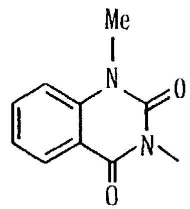 | $-CH_2-CH_2-O-$ | 4 | H | $CONH_2$ | H | $CH_2$ | OEt | 165-168 | 1.07(3H, t), 2.16(1H, dd), 2.35(1H, dd), 2.65-2.76(2H, m), 2.91-2.94(1H, m), 3.53(3H, s), 3.96(2H, q), 4.16(2H, t), 4.33 (2H, t), 6.69(1H, brs), 6.82-6.85(2H, m), 7.01-7.04(2H, m), 7.23(1H, brs), 7.31(1H, ddd), 7.45(1H, dd), 7.78(1H, ddd), 8.07(1H, dd) ($DMSO-d_6$) |
| 1AJ | 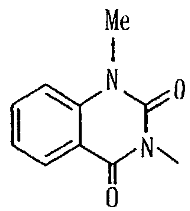 | $-CH_2-CH_2-O-$ | 4 | H | $CH_2CONH_2$ | H | O | OEt | 142-144 | 1.29(3H, t), 2.89(1H, dd), 3.10(1H, dd), 3.61(3H, s), 3.79(1H, d), 4.03(1H, d), 4.20-4.29(2H, m), 4.32(2H, t), 4.52(2H, t), 5.68 (1H, brs), 6.04(1H, brs), 6.87-6.90(2H, m), 7.14-7.18(2H, m), 7.22(1H, dd), 7.27(1H, ddd), 7.70(1H, ddd), 8.22(1H, dd) |
| 1AK | 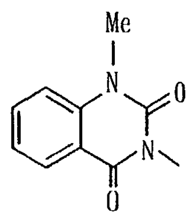 | $-CH_2-CH_2-O-$ | 4 | H | $CH_2CONH_2$ | H | O | $NH_2$ | 220-222 | 2.80(1H, dd), 2.96(1H, dd), 3.53(3H, s), 3.70-3.80(2H, m), 3.90(1H, dd), 4.16(2H, t), 4.33(2H, t), 6.82-6.86(2H, m), 6.90-7.18 (5H, m), 7.31(1H, ddd), 7.46(1H, dd), 7.52 (1H, brs), 7.79(1H, ddd), 8.07(1H, dd) ($DMSO-d_6$) |

Table 11

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1AL | 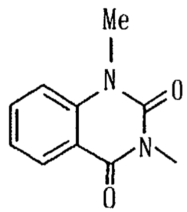 | $-CH_2-CH_2-O-$ | 4 | H | $CH_2COOEt$ | H | O | $NH_2$ | 138-139.5 | 1.17(3H, t), 2.81(1H, dd), 2.93(1H, dd), 3.53(3H, s), 3.97(1H, dd), 3.98(1H, d), 4.08(2H, q), 4.09(1H, d), 4.16(2H, t), 4.33 (2H, t), 6.81-6.85(2H, m), 7.11-7.22(4H, m), 7.31(1H, ddd), 7.45(1H, dd), 7.78(1H, ddd), 8.07(1H, dd) (DMSO-$d_6$) |
| 1AM | 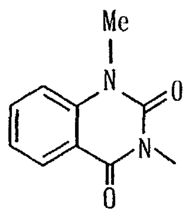 | $-CH_2-CH_2-O-$ | 4 | H | $CH_2CH_2OH$ | H | O | $NH_2$ | 144-146 | 2.74(1H, dd), 2.91(1H, dd), 3.35-3.50 (4H, m), 3.53(3H, s), 3.81(1H, dd), 4.16 (2H, t), 4.33(2H, t), 4.62(1H, t), 6.81-6.85 (2H, m), 7.10-7.13(3H, m), 7.24(1H, brs), 7.31(1H, ddd), 7.45(1H, dd), 7.78(1H, ddd), 8.07(1H, dd) (DMSO-$d_6$) |
| 1AN | 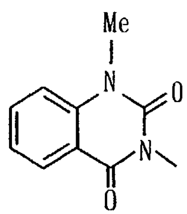 | $-CH_2-CH_2-O-$ | 4 | H | COOMe | H | $CH_2$ | OMe | 90-92 | 2.39(1H, dd), 2.52(1H, dd), 2.69(1H, dd), 2.81(1H, dd), 2.93-2.98(1H, m), 3.53(3H, s), 3.54(3H, s), 3.55(3H, s), 4.17(2H, t), 4.33 (2H, t), 6.83-6.86(2H, m), 7.02-7.05(2H, m), 7.31(1H, ddd), 7.45(1H, dd), 7.78(1H, ddd), 8.07(1H, dd) (DMSO-$d_6$) |
| 1AO | 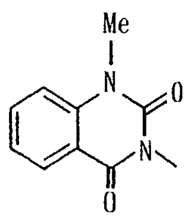 | $-CH_2-CH_2-O-$ | 4 | H | $CH_2COOMe$ | H | O | OMe | 102-103.5 | 2.87-2.97(2H, m), 3.53(3H, s), 3.59(3H, s), 3.61(3H, s), 4.08(1H, d), 4.16(2H, t), 4.19 (1H, d), 4.26(1H, t), 4.33(2H, t), 6.82-6.85 (2H, m), 7.08-7.11(2H, m), 7.31(1H, ddd), 7.45(1H, dd), 7.78(1H, ddd) 8.07(1H, dd) (DMSO-$d_6$) |

Table 12

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1AP | 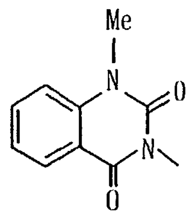 | $-CH_2-CH_2-O-$ | 4 | H | COOEt | H | $CH_2$ | OEt | oil | 1.09(3H, t), 1.14(3H, t), 2.37(1H, dd), 2.49(1H, dd), 2.69(1H, dd), 2.80(1H, dd), 2.91-2.95(1H, m), 3.53(3H, s), 2.97-4.04 (4H, m), 4.17(2H, t), 4.33(2H, t), 6.83-6.87 (2H, m), 7.03-7.06(2H, m), 7.31(1H, ddd), 7.45(1H, dd), 7.78(1H, ddd), 8.07(1H, dd) (DMSO-$d_6$) |
| 1AQ | 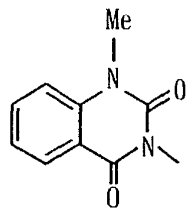 | $-CH_2-CH_2-O-$ | 4 | H | $CH_2COOEt$ | H | O | OEt | oil | 1.11(3H, t), 1.17(3H, t), 2.92(2H, d), 3.53(3H, s), 4.02-4.25(9H, m), 4.33(2H, t), 6.82-6.85(2H, m), 7.09-7.12(2H, m), 7.31(1H, ddd), 7.45(1H, dd), 7.78(1H, ddd), 8.07(1H, dd) |
| 1AR | 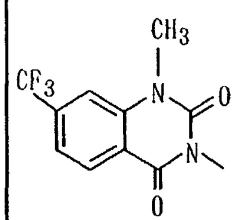 | $-CH_2-CH_2-O-$ | 4 | H | $CH_2CH_3$ | H | O | OH | 119-121 | 1.16(3H, t), 2.92(1H, dd), 3.06(1H, dd), 3.40-3.48(1H, m), 3.52-3.65(1H, m), 3.65 (3H, s), 4.03(1H, dd), 4.26(2H, t), 4.54 (2H, t), 6.81-6.85(2H, m), 7.09-7.14 (2H, m), 7.43(1H, s), 7.51(1H, d), 8.37(1H, d) |
| 1AS | 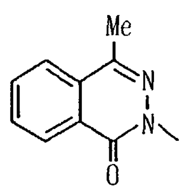 | $-CH_2-CH_2-O-$ | 4 | H | $CH_2COOEt$ | H | O | $NH_2$ | 116-118 | 1.16(3H, t), 2.55(3H, s), 2.80(1H, dd), 2.93(1H, dd), 3.97(1H, dd), 3.98(1H, d), 4.04(2H, q), 4.10(1H, d), 4.34(2H, t), 4.36 (2H, t), 6.80-6.84(2H, m), 7.09-7.14 (3H, m), 7.22(1H, brs), 7.84-7.95(3H, m), 8.30(1H, d)(DMSO-$d_6$) |

Table 13

| No. | W | A | PS | $R^1$ | $R^2$ | $R^3$ | X | $Y^1$ | m.p. (℃) | NMR($CDC\ell_3$, δ) |
|---|---|---|---|---|---|---|---|---|---|---|
| 1AT | 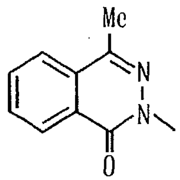 | $-CH_2-CH_2-O-$ | 4 | H | $CH_2CH_2OH$ | H | O | $NH_2$ | 127-128.5 | 2.56(3H, s), 2.73(1H, dd), 2.90(1H, dd), 3.35-3.47(4H, m), 3.80(1H, dd), 4.34(2H, t), 4.46(2H, t), 4.62(1H, t), 6.80-6.83(2H, m), 7.09-7.12(3H, m), 7.24(1H, brs), 7.85-7.95(3H, m), 8.30(1H, d) (DMSO-$d_6$) |
| 1AU | 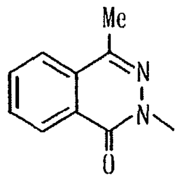 | $-CH_2-CH_2-O-$ | 4 | H | $CH_2COOMe$ | H | O | OMe | oil | 2.55(3H, s), 2.90-2.92(2H, m), 3.58(3H, s), 3.61(3H, s), 4.08(1H, d), 4.19(1H, d), 4.25(1H, t), 4.34(2H, t), 4.46(2H, t), 6.81-6.84(2H, m), 7.07-7.10(2H, m), 7.84-7.95 (3H, m), 8.28-8.31(1H, m) (DMSO-$d_6$) |
| 1AV | 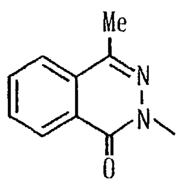 | $-CH_2-CH_2-O-$ | 4 | H | $CH_2COOEt$ | H | O | OEt | oil | 1.10(3H, t), 1.16(3H, t), 2.55(3H, t), 2.91(2H, d), 4.01-4.11(5H, m), 4.17(1H, d), 4.22(1H, t), 4.34(2H, t), 4.46(2H, t), 6.81-6.84(2H, m), 7.07-7.11(2H, m), 7.85-7.89(1H, m), 7.94-7.95(2H, m), 8.29-8.31 (1H, m) (DMSO-$d_6$) |
| 1AW | 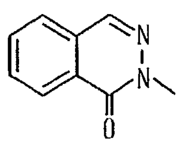 | $-CH_2-CH_2-O-$ | 4 | H | 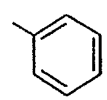 | H | O | OMe | 104-105 | 3.12-3.20(2H, m), 3.69(3H, s), 4.39(2H, t), 4.63(2H, t), 4.71-4.75(1H, m), 6.79-6.96 (5H, m), 7.15-7.26(4H, m), 7.67-7.83(3H, m), 8.17(1H, s), 8.42-8.45 (1H, m) |

Table 14

| No. | W | A | PS | $R^1$ | $R^2$ | $R^3$ | X | $Y^1$ | m.p. (℃) | NMR($CDCl_3$, δ) |
|---|---|---|---|---|---|---|---|---|---|---|
| 1AX | 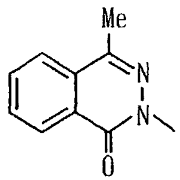 | $-CH_2-CH_2-O-$ | 4 | H | 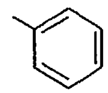 | H | O | OMe | 138-140 | 2.58(3H, s), 3.10-3.20(2H, m), 3.69(3H, s), 4.38(2H, t), 4.59(2H, t), 4.72-4.75(1H, m), 6.79-6.96(5H, m), 7.15-7.26(4H, m), 7.73-7.83(3H, m), 8.45-8.48(1H, m) |
| 1AY | 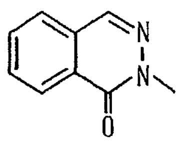 | $-CH_2-CH_2-O-$ | 4 | H | 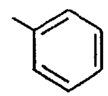 | H | O | OEt | oil | 1.16(3H, t), 3.15(2H, d), 4.11-4.71(7H, m), 6.76-7.23(9H, m), 7.67-7.76(3H, m), 8.15(1H, s), 8.30-8.50(1H, m) |
| 1AZ | 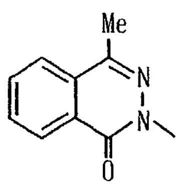 | $-CH_2-CH_2-O-$ | 4 | H | 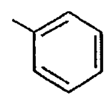 | H | O | OEt | oil | 1.17(3H, t), 2.56(3H, s), 3.15(2H, d), 4.03-4.78(7H, m), 6.76-7.26(9H, m), 7.70-7.78(3H, m), 8.40-8.50(1H, m) |
| 1BA | 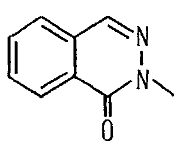 | $-CH_2-CH_2-O-$ | 4 | H | 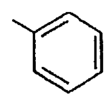 | H | O | OH | 60-62 | 3.19(2H, d), 4.37(2H, t), 4.63(2H, t), 4.78(1H, t), 5.50(1H, br), 6.82-6.97(5H, m), 7.20-7.26(4H, m), 7.67-7.83(3H, m), 8.18(1H, s), 8.42-8.44(1H, m) |

Table 15

| No. | W | A | PS | $R^1$ | $R^2$ | $R^3$ | X | $Y^1$ | m.p. (°C) | NMR(CDC $\ell_3$, δ) |
|---|---|---|---|---|---|---|---|---|---|---|
| 1BB | 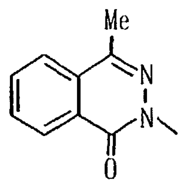 | $-CH_2-CH_2-O-$ | 4 | H | 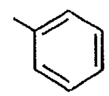 | H | O | OH | 155-159 | 2.57(3H, s), 3.18(2H, d), 4.35(2H, t), 4.58(2H, t), 4.76(1H, t), 6.82-6.95(5H, m), 7.16-7.26(4H, m), 7.72-7.80(3H, m), 8.44-8.46(1H, m) |
| 1BC | 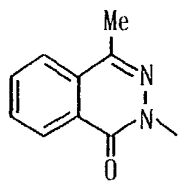 | $-CH_2-CH_2-O-$ | 4 | H | $CH_2CH_3$ | H | O | OH | 107-109 | 1.15(3H, t), 2.59(3H, s), 2.89-3.06(2H, m), 3.36-3.60(2H, m), 4.01-4.03(1H, m), 4.38(2H, t), 4.60(2H, t), 6.84-6.87(2H, m), 7.11-7.14(2H, m), 7.74-7.84(3H, m), 8.46-8.48(1H, m) |
| 1BD | 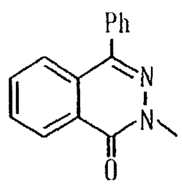 | $-CH_2-CH_2-O-$ | 4 | H | $CH_2CH_3$ | H | O | OH | 114-118 | 1.14(3H, t), 2.88-3.06(2H, m), 3.36-3.61 (2H, m), 3.99-4.02(1H, m), 4.44(2H, t), 4.71(2H, t), 6.85(2H, d), 7.12(2H, d), 7.52-7.59(5H, m), 7.72-7.81(3H, m), 8.53-8.55(1H, m) |
| 1BE | 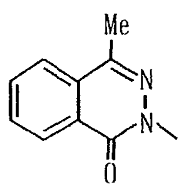 | $-CH_2-CH_2-O-$ | 4 | COOEt | 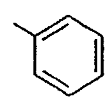 | H | O | OEt | 87-88 | 1.14(6H, t), 2.61(3H, s), 3.56(2H, s), 4.16(4H, q), 4.39(2H, t), 4.61(2H, t), 6.82-6.84(2H, m), 6.97-7.04(3H, m), 7.09-7.11(2H, m), 7.24-7.29(2H, m), 7.77-7.84(3H, m), 8.48-8.50(1H, m) |

Table 16

| No. | W | A | PS | $R^1$ | $R^2$ | $R^3$ | X | $Y^1$ | m.p. (°C) | NMR ($CDCl_3$, δ) |
|---|---|---|---|---|---|---|---|---|---|---|
| 1BF | 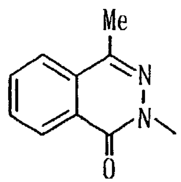 | $-CH_2-CH_2-O-$ | 4 | COOEt | 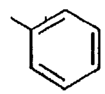 | H | O | OH | 70-73 | 1.04(3H, t), 2.59(3H, s), 3.53(2H, s), 4.09-4.18(2H, m), 4.35(2H, t), 4.57(2H, t), 6.30(1H, br), 6.73-7.26(9H, m), 7.73-7.83(3H, m), 8.42-8.44(1H, m) |
| 1BG | 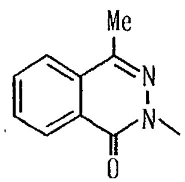 | $-CH_2-CH_2-O-$ | 3 | H | $CH_2CH_3$ | H | O | OH | 120-122 | 1.16(3H, t), 2.61(3H, s), 2.95-3.09(2H, m), 3.41-3.62(2H, m), 4.06-4.09(1H, m), 4.41-4.44(2H, m), 4.57-4.61(2H, m), 6.80-6.86(3H, m), 7.15-7.19(1H, m), 7.76-7.85(3H, m), 8.46-8.48(1H, m) |
| 1BH | 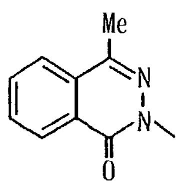 | $-CH_2-CH_2-O-$ | 3 | H | $CH_2CH_3$ | H | O | $NH_2$ | 91-92 | 1.11(3H, t), 2.60(3H, s), 2.83-2.88(1H, m), 3.07-3.11(1H, m), 3.36-3.53(2H, m), 3.90-3.93(1H, m), 4.40(2H, t), 4.60(2H, t), 5.46(1H, br), 6.48(1H, br), 6.79-6.84(3H, m), 7.14-7.18(1H, m), 7.75-7.84(1H, m), 8.46-8.48(1H, m) |
| 1BI | 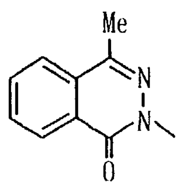 | $-CH_2-CH_2-O-$ | 4 | H | $CH_2CH_3$ | H | O | OEt | 54-56 | 1.15(3H, t), 1.21(3H, t), 2.60(3H, s), 2.91-2.93(2H, m), 3.31-3.35(1H, m), 3.56-3.60(1H, m), 3.92-3.96(1H, m), 4.15(2H, q), 4.39(2H, t), 4.60(2H, t), 6.85(2H, d), 7.12(2H, d), 7.75-7.84(3H, m), 8.46-8.48(1H, m) |

Table 17

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1BJ | 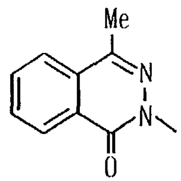 | $-CH_2-CH_2-O-$ | 4 | H | $CH_2CF_3$ | H | O | OH | 152-154 | 2.60(3H, s), 3.00-3.11(2H, m), 3.67-3.72(1H, m), 3.95-4.00(1H, m), 4.17-4.20(1H, m), 4.39(2H, t), 4.60(2H, t), 6.85(2H, d), 7.13(2H, d), 7.76-7.83(3H, m), 8.46-8.48(1H, m) |
| 1BK | 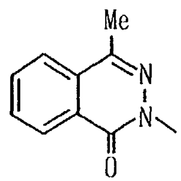 | $-CH_2-CH_2-O-$ | 4 | H | $CH_2CH_2OCH_3$ | H | O | OH | oil | 2.60(3H, s), 2.84-2.90(1H, m), 3.12-3.17(1H, m), 3.39(3H, s), 3.39-3.55(4H, m), 4.02-4.05(1H, m), 4.39(2H, t), 4.60(2H, t), 6.86(2H, d), 7.14(2H, d), 7.76-7.83(3H, m), 8.46-8.48(1H, m) |
| 1BL | 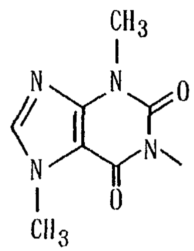 | $-CH_2-CH_2-O-$ | 4 | H | $CH_2CH_3$ | H | O | OH | amorphous | 1.16(3H, t), 2.90-3.08(2H, m), 3.41-3.47(1H, m), 3.55-3.59(1H, m), 3.57(3H, s), 3.99(3H, s), 3.99-4.04(1H, m), 4.21(2H, t), 4.45(2H, t), 6.85(2H, d), 7.13(2H, d), 7.55(1H, s) |
| 1BM | 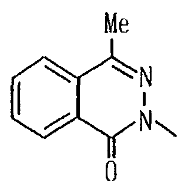 | $-CH_2-CH_2-O-$ | 4 | H | $CH_2CH_2OCH_3$ | H | O | OMe | 67-68 | 2.59(3H, s), 2.94-2.96(2H, m), 3.29(3H, s), 3.45-3.49(3H, m), 3.66-3.68(1H, m), 3.68(3H, s), 4.04-4.08(1H, m), 4.38(2H, t), 4.60(2H, t), 6.85(2H, d), 7.06(2H, d), 7.75-7.82(3H, m), 8.46-8.48(1H, m) |

Table 18

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1BN | 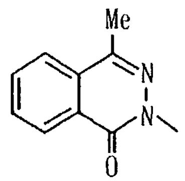 | $-CH_2-CH_2-O-$ | 4 | H | $CH_2CF_3$ | H | O | OMe | 111-112 | 2.59(3H, s), 2.93-3.06(2H, m), 3.62-3.72(1H, m), 3.72(3H, s), 3.93-4.03(1H, m), 4.13-4.16(1H, m), 4.39(2H, t), 4.60(2H, t), 6.85(2H, d), 7.10(2H, d), 7.74-7.83(3H, m), 8.46-8.48(1H, m) |
| 1BO | 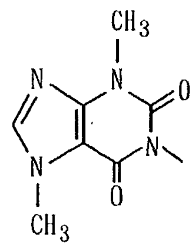 | $-CH_2-CH_2-O-$ | 4 | H | $CH_2CH_3$ | H | O | OMe | 88-89 | 1.15(3H, t), 2.93(2H, d), 3.23-3.58(2H, m), 3.58(3H, s), 3.69(3H, s), 3.89-4.09(1H, m), 4.00(3H, s), 4.05-4.55(4H, m), 6.82(2H, d), 7.12(2H, d), 7.52(1H, s) |
| 1BP | 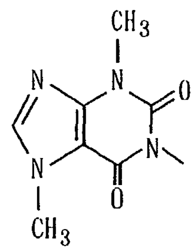 | $-CH_2-CH_2-O-$ | 4 | H | $CH_2CH_3$ | H | O | $NH_2$ | oil | 1.12(3H, t), 2.81-2.87(1H, m), 3.04-3.08(1H, m), 3.38-3.53(2H, m), 3.58(3H, s), 3.85-3.88(1H, m), 3.99(3H, s), 4.21(2H, t), 4.44(2H, t), 5.58(1H, br), 6.43(1H, br), 6.84(2H, d), 7.13(2H, d), 7.52(1H, s) |
| 1BQ | 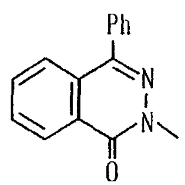 | $-CH_2-CH_2-O-$ | 4 | H | $CH_2CH_3$ | H | O | OEt | oil | 1.06-1.28(6H, m), 2.92(2H, d), 3.15-3.75(2H, m), 3.86-4.26(3H, m), 4.40-4.80(4H, m), 6.79-7.17(4H, m), 7.49-7.80(8H, m), 8.45-8.60(1H, m) |

Table 19

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1BR | 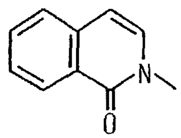 | $-CH_2-CH_2-O-$ | 4 | H | $CH_2CH_3$ | H | O | $NH_2$ | 143-145 | 1.02(3H, t), 2.71(1H, dd), 2.85(1H, dd), 3.21-3.30(1H, m), 3.39-3.48(1H, m), 3.73(1H, dd), 4.25(2H, t), 4.33(2H, t), 6.62(1H, d), 6.82-6.85(2H, m), 7.03-7.11 (4H, m), 7.47-7.52(2H, m), 7.63-7.72(2H, m) 8.23(1H, d)(DMSO-$d_6$) |
| 1BS | 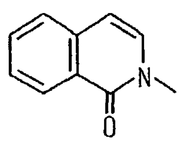 | $-CH_2-CH_2-O-$ | 4 | H | $CH_2CH_3$ | H | O | OH | 79-80 | 1.02(3H, t), 2.78(1H, dd), 2.87(1H, dd), 3.21-3.33(1H, m), 3.46-3.57(1H, m), 3.91(1H, dd), 4.25(2H, t), 4.34(2H, t), 6.62(1H, d), 6.82-6.86(2H, m), 7.09-7.13(2H, m), 7.48-7.52(2H, m), 7.63-7.72(2H, m), 8.24(1H, dd), 12.25(1H, br)(DMSO-$d_6$) |
| 1BT | 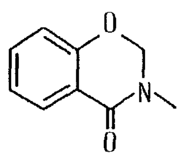 | $-CH_2-CH_2-O-$ | 4 | H | $CH_2CF_3$ | H | O | OMe | 105-106 | 2.99-3.04(2H, m), 3.64-3.73(4H, m), 3.94-4.02(3H, m), 4.14-4.19(3H, m), 5.36(2H, s), 6.80(2H, d), 6.97(1H, dd), 7.09-7.14(3H, m), 7.44(1H, ddd), 7.95(1H, dd) |
| 1BU | 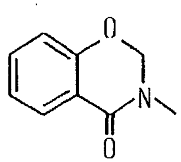 | $-CH_2-CH_2-O-$ | 4 | H | $CH_2CF_3$ | H | O | OH | 105-107 | 3.01(1H, dd), 3.11(1H, dd), 3.67-3.72(1H, m), 3.93-4.00(3H, m), 4.14-4.21(3H, m), 5.36(2H, s), 6.80(2H, d), 6.97(1H, dd), 7.10(1H, ddd), 7.16(2H, d), 7.44(1H, ddd), 7.94(1H, dd) |

Table 20

| No. | W | A | PS | $R^1$ | $R^2$ | $R^3$ | X | $Y^1$ | m. p. (℃) | NMR($CDCl_3$, δ) |
|---|---|---|---|---|---|---|---|---|---|---|
| 1BV | 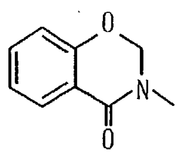 | $-CH_2-CH_2-O-$ | 4 | H | $CH_2CH_2OCH_3$ | H | O | OH | 94-96 | 2.90(1H, dd), 3.16(1H, dd), 3.37-3.47(5H, m), 3.53-3.56(1H, m), 3.66-3.67(1H, m), 3.95(2H, t), 4.04(1H, dd), 4.18(2H, t), 5.37(2H, s), 6.81(2H, d), 6.97(1H, dd), 7.10(1H, ddd), 7.17(2H, d), 7.44(1H, ddd), 7.95(1H, dd) |
| 1BW | 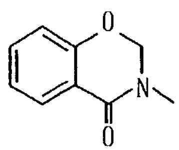 | $-CH_2-CH_2-O-$ | 4 | H | $CH_2CH_2OCH_3$ | H | O | OMe | 87-88 | 2.95-2.98(2H, m), 3.30(3H, s), 3.45-3.50 (3H, m), 3.67-3.71(4H, m), 3.95(2H, t), 4.07(1H, dd), 4.18(2H, t), 5.37(2H, s), 6.79(2H, d), 6.97(1H, d), 7.09-7.15(3H, m), 7.44(1H, ddd), 7.95(1H, dd) |

The following compounds can be prepared in accordance with processes similar to the processes described in the above Preparation Examples and Examples.

2-Methoxy-3-{4-[2-(1-oxo-1,2-dihydrophthalazin-2-yl)ethoxy]phenyl}propanamide;
2-Methylthio-3-{4-[2-(1-oxo-1,2-dihydrophthalazin-2-yl)ethoxy]phenyl}propanamide;
2-Propylthio-3-{4-[3-(1-oxo-1,2-dihydrophthalazin-2-yl)propoxy]phenyl}propanamide;
2-Propoxy-3-{4-[2-(1-oxo-1,2-dihydrophthalazin-2-yl)ethoxy]phenyl}propanamide;
2-Propoxy-3-{4-[3-(1-oxo-1,2-dihydrophthalazin-2-yl)propoxy]phenyl}propanamide;
2-Isopropoxy-3-{4-[2-(1-oxo-1,2-dihydrophthalazin-2-yl)ethoxy]phenyl}propanamide;
2-Isopropoxy-3-{4-[3-(1-oxo-1,2-dihydrophthalazin-2-yl)propyl]phenyl}propanamide;
2-Phenoxy-3-{4-[2-(1-oxo-1,2-dihydrophthalazin-2-yl)ethoxy]phenyl}propanamide;
2-Phenylthio-3-{4-[2-(1-oxo-1,2-dihydrophthalazin-2-yl)ethoxy]phenyl}propanamide;
2-Methylamino-3-{4-[2-(1-oxo-1,2-dihydrophthalazin-2-yl)ethoxy]phenyl}propanamide;
2-Ethylamino-3-{4-[2-(1-oxo-1,2-dihydrophthalazin-2-yl)ethoxy]phenyl}propanamide;
2-Methyl-2-propoxy-3-{4-[2-(1-oxo-1,2-dihydro-phthalazin-2-yl)ethoxy]phenyl}propanamide;
2-Methy-2-propylthio-3-{4-[2-(1-oxo-1,2-dihydro-phthalazin-2-yl)ethoxy]phenyl}propanamide;
2-Methyl-2-propylamino-3-{4-[2-(1-oxo-1,2-dihydro-phthalazin-2-yl)ethoxy]phenyl}propanamide;
2-Methyl-2-isopropoxy-3-{4-[3-(1-oxo-1,2-dihydro-phthalazin-2-yl)propyl]phenyl}propanamide;
2-Ethoxy-3-{4-[2-(6-methyl-1-oxo-1,2-dihydro-phthalazin-2-yl)ethoxy]phenyl}propanamide;
2-butoxy-3-{4-[2-(4,6-dimethyl-1-oxo-1,2-dihydro-phthalazin-2-yl)ethoxy]phenyl}propanamide;
2-Ethoxy-3-{4-[2-(6-methoxy-4-phenyl-1-oxo-1,2-dihydrophthalazin-2-yl)propoxy]phenyl}propanamide;
2-Ethylthio-3-{4-[2-(6-methoxy-4-phenyl-1-oxo-1,2-dihydrophthalazin-2-yl)propoxy]phenyl}propanamide;
2-Ethylamino-3-{4-[2-(6-methoxy-4-phenyl-1-oxo-1,2-dihydrophthalazin-2-yl)propoxy]phenyl}propanamide;
2-Ethoxy-3-{4-[2-(6-methoxy-4-(2-pyridyl)-1-oxo-1,2-dihydrophthalazin-2-yl)propoxy]phenyl}propanamide;
2-Methylthio-3-{4-[2-(6-methoxy-4-(2-pyridyl)-1-oxo-1,2-dihydrophthalazin-2-yl)ethoxy]phenyl}propanamide;
2-Methylamino-3-{4-[2-(6-methoxy-4-(2-pyridyl)-1-oxo-1,2-dihydrophthalazin-2-yl)ethoxy]phenyl}propanamide;
2-Ethoxy-3-{4-[2-(6-methoxy-4-(4-pyridyl)-1-oxo-1,2-dihydrophthalazin-2-yl)ethoxy]phenyl}propanamide;
2-Ethoxy-3-{4-[3-(4-ethyl-6-methoxy-1-oxo-1,2-dihydrophthalazin-2-yl)butoxy]phenyl}propanamide;
N1-Ethyl-2-{4-[2-(1-oxo-1,2-dihydrophthalazin-2-yl)-1-methylethoxy]-3-methylbenzyl}-2-ethoxybutanamide;
6-Chloro-2-{3-[4-(2-ethoxy-2-methyl-3-oxo-3-piperidinopropyl)phenyl]-2-methyl-3-oxopropyl}-1,2-dihydro-1-phthalazinone;
7-Methyl-2-{3-[4-(2-ethoxy-2-methyl-3-oxo-3-pyrrolidinopropyl)phenyl]-2-hydroxypropyl}-1,2-dihydro-1-phthalazinone;
Ethyl 2-methylthio-3-{4-[2-(1-oxo-1,2-dihydro-phthalazin-2-yl)ethoxy]phenyl}propanoate;
Methyl 2-isopropoxy-3-{4-[3-(1-oxo-1,2-dihydro-phthalazin-2-yl)propyl]phenyl}propanoate;
Propyl 2-phenoxy-3-{4-[2-(1-oxo-1,2-dihydro-phthalazin-2-yl)ethoxy]phenyl}propanoate;
Isopropyl 2-ethylamino-3-{4-[2-(1-oxo-1,2-dihydro-phthalazin-2-yl)ethoxy]phenyl}propanoate;
2-Isopropoxy-3-{4-[3-(1-oxo-1,2-dihydrophthalazin-2-yl)propyl]phenyl}propanoic acid;
2-Isopropylthio-3-{4-[2-(1-oxo-1,2-dihydro-phthalazin-2-yl)ethoxy]phenyl}propanoic acid;
2-Isopropylamino-3-{4-[2-(1-oxo-1,2-dihydro-phthalazin-2-yl)ethoxy]phenyl}propanoic acid;
2-Isopropoxy-3-{4-[4-(1-oxo-1,2-dihydrophthalazin-2-yl)butyl]phenyl}propanoic acid;
2-Phenoxy-3-{4-[2-(1-oxo-1,2-dihydrophthalazin-2-yl)propoxy]phenyl}propanoic acid;
2-Propoxy-3-{4-[2-(5-methyl-4-oxo-3,4-dihydro-2H-1,3-benzoxazin-3-yl)ethoxy]phenyl}propanamide;
2-Ethylthio-3-{4-[2-(5-methyl-4-oxo-3,4-dihydro-2H-1,3-benzoxazin-3-yl)ethoxy]phenyl}propanamide;
2-Ethylamino-3-{4-[2-(5-methyl-4-oxo-3,4-dihydro-2H-1,3-benzoxazin-3-yl)ethoxy]phenyl}propanamide;
2-Ethoxy-3-{4-[2-(6-acetyl-4-oxo-3,4-dihydro-2H-1,3-benzoxazin-3-yl)ethoxy]phenyl}propanamide;
2-Butoxy-3-{4-[2-(6-butyryl-4-oxo-3,4-dihydro-2H-1,3-benzoxazin-3-yl)ethoxy]phenyl}propanamide;
2-Ethylthio-3-{4-[2-(6-amino-4-oxo-3,4-dihydro-2H-1,3-benzoxazin-3-yl)ethoxy]phenyl}propanamide;
2-Ethoxy-3-(4-[2-(6-methoxy-4-oxo-3,4-dihydro-2H-1,3-benzoxazin-3-yl)propoxy]phenyl}propanamide;
2-Ethoxy-3-(4-[3-(7-methoxy-4-oxo-3,4-dihydro-2H-1,3-benzoxazin-3-yl)propyl]phenyl}propanamide;
2-Ethoxy-3-{4-[3-(7-methyl-4-oxo-3,4-dihydro-2H-1,3-benzoxazin-3-yl)propyl]phenyl}propanamide;
2-Phenoxy-3-{4-[3-(7-methyl-4-oxo-3,4-dihydro-2H-1,3-benzoxazin-3-yl)propyl]phenyl}propanamide;
Ethyl 2-ethoxy-3-{4-[3-(4-oxo-3,4-dihydro-2H-1,3-benzoxazin-3-yl)propyl]phenyl}propanoate;
Propyl 2-methoxy-3-{4-[2-(4-oxo-3,4-dihydro-2H-1,3-benzoxazin-3-yl)ethoxy]phenyl}propanoate;
Ethyl 2-phenoxy-3-(4-[3-(4-oxo-3,4-dihydro-2H-1,3-benzoxazin-3-yl)propyl]phenyl}propanoate;
2-Ethoxy-3-{4-[3-(7-methyl-4-oxo-3,4-dihydro-2H-1,3-benzoxazin-3-yl)propyl]phenyl}propanoic acid;
2-Ethoxy-3-{4-[3-(7-chloro-4-oxo-3,4-dihydro-2H-1,3-benzoxazin-3-yl)propoxy]phenyl}propanoic acid;
2-Butoxy-3-{4-[4-(7-chloro-4-oxo-3,4-dihydro-2H-1,3-benzoxazin-3-yl)butoxy]phenyl}propanoic acid;

2-Benzyloxy-3-{4-[3-(7-cyano-4-oxo-3,4-dihydro-2H-1,3-benzoxazin-3-yl)butoxy]phenyl}propanoic acid;
N1-Ethyl-3-{4-[4-(7-cyano-4-oxo-3,4-dihydro-2H-1,3-benzoxazin-3-yl)butoxy]phenyl}-2-ethoxy-2-methyl-propanamide;
N1,N1-Dimethyl-3-{4-[2-(7-benzyloxy-4-oxo-3,4-dihydro-2H-1,3-benzoxazin-3-yl)ethoxy]phenyl}-2-ethoxy-2-methylpropanamide;
N1,N1-Diethyl-3-{4-[4-(7-benzyloxy-4-oxo-3,4-dihydro-2H-1,3-benzoxazin-3-yl)butoxy]phenyly-2-ethoxy-2-methylpropanamide;
6-Chloro-3-{3-[4-(2-ethoxy-2-methyl-3-oxo-3-piperidinopropyl)phenyl]-2-methyl-3-oxopropyl}-4-oxo-3,4-dihydro-2H-1,3-benzoxazine;
2-Ethoxy-3-{4-[2-(6-methyl-4-oxo-1-phenyl-1,2,3,4-tetrahydroquinazolin-3-yl)ethoxy]phenyl}propanamide;
2-Ethylthio-3-{4-[2-(6-methyl-4-oxo-1-phenyl-1,2,3,4-tetrahydroquinazolin-3-yl)ethoxy]phenyl}-propanamide;
2-Methoxy-3-{4-[2-(1,6-dimethyl-4-oxo-1,2,3,4-tetrahydroquinazolin-3-yl)ethoxy]phenyl}propanamide;
2-Propylamino-3-{4-[3-(1,6-dimethyl-4-oxo-1,2,3,4-tetrahydroquinazolin-3-yl)propyl]phenyl}propanamide;
2-Propylthio-3-{4-[3-(1,6-dimethyl-4-oxo-1,2,3,4-tetrahydroquinazolin-3-yl)propyl]phenyl}propanamide;
2-Ethoxy-3-{4-[3-(1-ethyl-6-methoxy-4-oxo-1,2,3,4-tetrahydroquinazolin-3-yl)propyl]phenyl}propanamide;
2-Isopropylthio-3-{4-[2-(1-ethyl-6-methoxy-4-oxo-1,2,3,4-tetrahydroquinazolin-3-yl)ethoxy]phenyl}-propanamide;
N1,N1-Dimethyl-2-(4-[2-(1-methyl-4-oxo-1,2,3,4-tetrahydroquinazolin-3-yl)ethoxy]benzyl}-2-ethoxy-butanamide;
N1,N1-Dimethyl-2-{4-[2-(1-methyl-4-oxo-1,2,3,4-tetrahydroquinazolin-3-yl)ethoxy]benzyl}-2-ethylthio-butanamide;
Methyl 2-ethoxy-3-{4-[2-(6-methyl-4-oxo-1-phenyl-1,2,3,4-tetrahydroquinazolin-3-yl)ethoxy]phenyl}-propanoate;
Methyl 2-ethoxy-3-{4-[2-(1,6-dimethyl-4-oxo-1-phenyl-1,2,3,4-tetrahydroquinazolin-3-yl)ethoxy]phenyl}-propanoate;
Isopropyl 2-ethylthio-3-{4-[2-(1-ethyl-6,7-dimethyl-4-oxo-1,2,3,4-tetrahydroquinazolin-3-yl)ethoxy]phenyl}-propanoate;
Ethyl 2-ethylamino-3-{4-[2-(1-ethyl-6-methyl-4-oxo-1,2,3,4-tetrahydroquinazolin-3-yl)propoxy]phenyl}-propanoate;
2-Ethoxy-3-{4-[3-(1-ethyl-6-methyl-4-oxo-1,2,3,4-tetrahydroquinazolin-3-yl)propyl]phenyl}propanoic acid;
2-Ethylthio-3-{4-[3-(1-ethyl-6-methyl-4-oxo-1,2,3,4-tetrahydroquinazolin-3-yl)propyl]phenyl}propanoic acid;
2-Ethylthio-3-{4-[2-(1-ethyl-6,7-dimethyl-4-oxo-1,2,3,4-tetrahydroquinazolin-3-yl)ethoxy]phenyl}propanoic acid;
2-Methoxy-3-{4-[2-(1-ethyl-7-methoxy-4-oxo-1,2,3,4-tetrahydroquinazolin-3-yl)ethoxy]phenyl}propanoic acid;
2-Propoxy-3-{4-[2-(1-ethyl-8-methyl-4-oxo-1,2,3,4-tetrahydroquinazolin-3-yl)ethoxy]phenyl}propanoic acid;
2-Isopropoxy-3-{4-[4-(1-ethyl-6-methyl-4-oxo-1,2,3,4-tetrahydroquinazolin-3-yl)butoxy]phenyl}propanoic acid;
2-Ethylthio-3-{4-[2-(1-ethyl-6-methyl-4-oxo-1,2,3,4-tetrahydroquinazolin-3-yl)propoxy]phenyl}propanoic acid;
2-Ethylamino-3-{4-[2-(1-ethyl-6-methyl-4-oxo-1,2,3,4-tetrahydroquinazolin-3-yl)propoxy]phenyl}propanoic acid;
2-Ethoxy-3-{4-[4-(1,7-dimethyl-4-oxo-1,2,3,4-tetrahydroquinazolin-3-yl)butanoyl]phenyl}propanoic acid;
2-Ethoxy-3-(4-[3-(1,7-dimethyl-4-oxo-1,2,3,4-tetrahydroquinazolin-3-yl)propanoyl]phenyl}propanoic acid;
2-Ethylthio-3-{4-[4-(1,7-dimethyl-4-oxo-1,2,3,4-tetrahydroquinazolin-3-yl)butanoyl]phenyl}propanoic acid;
2-Ethylamino-3-(4-[4-(1,7-dimethyl-4-oxo-1,2,3,4-tetrahydroquinazolin-3-yl)butanoyl]phenyl}propanoic acid;
3-{4-[4-(2-Carboxy-2-ethoxybutyl)phenoxy]propyl}-7-methoxy-1-methyl-4-oxo-1,2,3,4-tetrahydro-6-quinazolinecarboxylic acid;
2-Isopropoxy-3-{4-[2-(4-oxo-3,4-dihydro-2H-1,3-benzthiazin-3-yl)ethoxy]phenyl}propanamide;
2-Ethylthio-3-{4-[2-(4-oxo-3,4-dihydro-2H-1,3-benzthiazin-3-yl)ethoxy]phenyl}propanamide;
2-Ethoxy-3-{4-[2-(6-methyl-4-oxo-3,4-dihydro-2H-1,3-benzthiazin-3-yl)ethoxy]phenyl}propanamide;
N1,2-Dimethyl-2-ethoxy-3-{4-[2-(4-oxo-3,4-dihydro-2H-1,3-benzthiazin-3-yl)ethoxy]phenyl}propanamide;
2-Ethoxy-3-{4-[3-(4-oxo-3,4-dihydro-2H-1,3-benzthiazin-3-yl)propyl]phenyl}propanamide;
2-Ethylamino-3-{4-[3-(4-oxo-3,4-dihydro-2H-1,3-benzthiazin-3-yl)propyl]phenyl}propanamide;
2-Ethoxy-3-{4-[2-(4-oxo-3,4-dihydro-2H-1,3-benzthiazin-3-yl)propyl]phenyl}propanoic acid;
2-Ethylthio-3-{4-[2-(4-oxo-3,4-dihydro-2H-1,3-benzthiazin-3-yl)ethoxy]phenyl}propanoic acid;
2-Ethylamino-3-{4-[2-(4-oxo-3,4-dihydro-2H-1,3-benzthiazin-3-yl)ethoxy]phenyl}propanoic acid;
2-Ethoxy-3-{4-[2-(1-oxo-1,2,3,4-tetrahydro-isoquinolin-2-yl)ethoxy]phenyl}propanamide;
2-Propoxy-3-(4-[2-(1-oxo-1,2,3,4-tetrahydro-isoquinolin-2-yl)ethoxy]phenyl}propanamide;
2-Ethoxy-3-{4-[3-(1-oxo-1,2,3,4-tetrahydro-isoquinolin-2-yl)propyl]phenyl}propanamide;
2-Ethylthio-3-(4-[2-(1-oxo-1,2,3,4-tetrahydro-isoquinolin-2-yl)ethoxy]phenyl}propanamide;
2-Ethylamino-3-{4-[2-(1-oxo-1,2,3,4-tetrahydro-isoquinolin-2-yl)ethoxy]phenyl}propanamide;
N1,2-dimethyl-2-ethoxy-3-{4-[2-(1-oxo-1,2,3,4-tetrahydroisoquinolin-2-yl)ethoxy]phenyl}propanamide;
Methyl 2-ethoxy-3-{4-[2-(1-oxo-1,2,3,4-tetrahydro-isoquinolin-2-yl)ethoxy]phenyl}propanoate;
Isopropyl 2-ethoxy-3-{4-[3-(1-oxo-1,2,3,4-tetrahydroisoquinolin-2-yl)propyl]phenyl}propanoate;
2-Ethoxy-3-{4-[2-(1-oxo-1,2,3,4-tetrahydro-isoquinolin-2-yl)ethoxy]phenyl}propanoic acid;

2-Methoxy-3-{4-[3-(1-oxo-1,2,3,4-tetrahydro-isoquinolin-2-yl)propyl]phenyl}propanoic acid;
2-Isopropoxy-3-{4-[2-(3-methyl-4-oxo-3,4-dihydro-quinazolin-2-yl)ethoxy]phenyl}propanamide;
2-Phenoxy-3-{4-[2-(3-methyl-4-oxo-3,4-dihydro-quinazolin-2-yl)ethoxy]phenyl}propanamide;
2-Propoxy-3-{4-[3-(3-ethyl-4-oxo-3,4-dihydro-quinazolin-2-yl)propyl)phenyl}propanamide;
2-Isopropylthio-3-{4-[2-(3-methyl-4-oxo-3,4-dihydro-quinazolin-2-yl)ethoxy]phenyl)propanamide;
2-Ethylamino-3-{4-[2-(3-methyl-4-oxo-3,4-dihydro-quinazolin-2-yl)ethoxy]phenyl}propanamide;
N1,2-Dimethyl-2-ethoxy-3-{4-[2-(3-methyl-4-oxo-3,4-dihydroquinazolin-2-yl)ethoxy]phenyl}propanamide;
Ethyl 2-isopropylthio-3-{4-[2-(3-methyl-4-oxo-3,4-dihydroquinazolin-2-yl)ethoxy]phenyl}propanoate;
Isopropyl 2-ethylamino-3-{4-[2-(3-methyl-4-oxo-3,4-dihydroquinazolin-2-yl)ethoxy]phenyl}propanoate;
2-Ethoxy-3-{4-[2-(3-methyl-4-oxo-3,4-dihydro-quinazolin-2-yl)ethoxy]phenyl}propanoic acid;
2-Ethoxy-3-{4-[3-(3-methyl-4-oxo-3,4-dihydro-quinazolin-2-yl)propoxy]phenyl}propanoic acid;
2-Isopropylthio-3-{4-[2-(3-methyl-4-oxo-3,4-dihydro-quinazolin-2-yl)ethoxy]phenyl}propanoic acid;
2-Ethylamino-3-{4-[2-(3-methyl-4-oxo-3,4-dihydro-quinazolin-2-yl)ethoxy]phenyl}propanoic acid;
2-Isopropoxy-3-{4-[2-(1-ethyl-2,4-dioxo-1,2,3,4-tetrahydroquinazolin-3-yl)ethoxy]phenyl}propanamide;
2-Methoxy-3-(4-[3-(1-methyl-2,4-dioxo-1,2,3,4-tetrahydroquinazolin-3-yl)propyl]phenyl}propanamide;
2-Isopropoxy-3-{4-[2-(6,7-dimethoxy-1-methyl-2,4-dioxo-1,2,3,4-tetrahydroquinazolin-3-yl)ethoxy]phenyl}-propanamide;
2-Isopropylthio-3-{4-[2-(1-methyl-2,4-dioxo-1,2,3,4-tetrahydroquinazolin-3-yl)ethoxy]phenyl}propanamide;
2-Isopropylamino-3-{4-[2-(1-methyl-2,4-dioxo-1,2,3,4-tetrahydroquinazolin-3-yl)ethoxy]phenyl}-propanamide;
N1,2-Dimethyl-2-ethoxy-3-{4-[2-(1-methyl-2,4-dioxo-1,2,3,4-tetrahydroquinazolin-3-yl)ethoxy]phenyl}-propanamide;
2-Isopropoxy-2-methyl-3-(4-[2-(1-methyl-2,4-dioxo-1,2,3,4-tetrahydroquinazolin-3-yl)ethoxy]phenyl}-propanamide;
Ethyl 2-isopropoxy-3-{4-[2-(1-methyl-2,4-dioxo-1,2,3,4-tetrahydroquinazolin-3-yl)ethoxy]phenyl}-propanoate;
Propyl 2-methoxy-3-(4-[3-(1-methyl-2,4-dioxo-1,2,3,4-tetrahydroquinazolin-3-yl)propyl]phenyl}-propanoate;
2-Isopropoxy-3-{4-[2-(1-methyl-2,4-dioxo-1,2,3,4-tetrahydroquinazolin-3-yl)ethoxy]phenyl}propanoic acid;
2-Methoxy-3-{4-[3-(1-methyl-2,4-dioxo-1,2,3,4-tetrahydroquinazolin-3-yl)propyl]phenyl}propanoic acid;
2-Isopropoxy-3-{4-[2-(6,7-dimethoxy-1-methyl-2,4-dioxo-1,2,3,4-tetrahydroquinazolin-3-yl)ethoxy]phenyl}-propanoic acid;
2-Isopropoxy-2-methyl-3-{4-[2-(1-methyl-2,4-dioxo-1,2,3,4-tetrahydroquinazolin-3-yl)ethoxy]phenyl}propanoic acid;
2-Isopropoxy-3-{4-[2-(2,4-dioxo-3,4-dihydro-2H-1,3-benzoxazin-3-yl)ethoxy]phenyl}propanamide;
2-Propoxy-3-{4-[3-(2,4-dioxo-3,4-dihydro-2H-1,3-benzoxazin-3-yl)propyl]phenyl}propanamide;
2-Ethoxy-2-methyl-3-{4-[3-(2,4-dioxo-3,4-dihydro-2H-1,3-benzoxazin-3-yl)propyl]phenyl}propanamide;
2-Ethylthio-3-{4-[3-(2,4-dioxo-3,4-dihydro-2H-1,3-benzoxazin-3-yl)propyl]phenyl}propanamide;
2-Ethylamino-3-{4-[3-(2,4-dioxo-3,4-dihydro-2H-1,3-benzoxazin-3-yl)propyl]phenyl}propanamide;
Methyl 2-isopropoxy-3-(4-[2-(2,4-dioxo-3,4-dihydro-2H-1,3-benzoxazin-3-yl)ethoxy]phenyl}propanoate;
Ethyl 2-ethoxy-2-methyl-3-{4-[3-(2,4-dioxo-3,4-dihydro-2H-1,3-benzoxazin-3-yl)propyl]phenyl}propanoate;
2-Isopropoxy-3-{4-[2-(2,4-dioxo-3,4-dihydro-2H-1,3-benzoxazin-3-yl)ethoxy]phenyl}propanoic acid;
2-Propoxy-3-{4-[3-(2,4-dioxo-3,4-dihydro-2H-1,3-benzoxazin-3-yl)propyl]phenyl}propanoic acid;
2-Ethoxy-2-methyl-3-{4-[3-(2,4-dioxo-3,4-dihydro-2H-1,3-benzoxazin-3-yl)propyl]phenyl}propanoic acid;
2-Ethylthio-3-{4-[3-(2,4-dioxo-3,4-dihydro-2H-1,3-benzoxazin-3-yl)propyl]phenyl}propanoic acid;
2-Ethylamino-3-{4-[3-(2,4-dioxo-3,4-dihydro-2H-1,3-benzoxazin-3-yl)propyl]phenyl}propanoic acid;
2-Ethoxy-3-{4-[2-(2,4-dioxo-3,4-dihydro-2H-1,3-benzthiazin-3-yl)ethoxy]phenyl}propanamide;
2-Ethoxy-3-(4-[3-(2,4-dioxo-3,4-dihydro-2H-1,3-benzthiazin-3-yl)propyl]phenyl}propanamide;
2-Ethylthio-3-{4-[2-(2,4-dioxo-3,4-dihydro-2H-1,3-benzthiazin-3-yl)ethoxy]phenyl}propanamide;
2-Ethylamino-3-{4-[2-(2,4-dioxo-3,4-dihydro-2H-1,3-benzthiazin-3-yl)ethoxy]phenyl}propanamide;
N1,2-Dimethyl-2-ethoxy-3-{4-[2-(2,4-dioxo-3,4-dihydro-2H-1,3-benzthiazin-3-yl)ethoxy]phenyl}propanamide;
Methyl 2-ethoxy-3-{4-[2-(2,4-dioxo-3,4-dihydro-2H-1,3-benzthiazin-3-yl)ethoxy]phenyl}propanoate;
Propyl 2-ethylthio-3-{4-[2-(2,4-dioxo-3,4-dihydro-2H-1,3-benzthiazin-3-yl)ethoxy]phenyl}propanoate;
2-Ethoxy-3-{4-[2-(2,4-dioxo-3,4-dihydro-2H-1,3-benzthiazin-3-yl)ethoxy]phenyl}propanoic acid;
2-Ethoxy-3-{4-[3-(2,4-dioxo-3,4-dihydro-2H-1,3-benzthiazin-3-yl)propyl]phenyl}propanoic acid;
2-Ethylthio-3-{4-[2-(2,4-dioxo-3,4-dihydro-2H-1,3-benzthiazin-3-yl)ethoxy]phenyl}propanoic acid;
2-Ethylamino-3-(4-[2-(2,4-dioxo-3,4-dihydro-2H-1,3-benzthiazin-3-yl)ethoxy]phenyl}propanoic acid;
2-Isopropoxy-3-{4-[2-(4-oxo-3,4-dihydroquinazolin-3-yl)ethoxy]phenyl}propanamide;
2-Phenoxy-3-{4-[2-(4-oxo-3,4-dihydroquinazolin-3-yl)ethoxy]phenyl}propanamide;
2-Methyl-2-propoxy-3-{4-[3-(4-oxo-3,4-dihydro-quinazolin-3-yl)propyl]phenyl}propanamide;
2-Isopropylthio-3-{4-[2-(4-oxo-3,4-dihydro-quinazolin-3-yl)ethoxy]phenyl}propanamide;

2-Ethylamino-3-{4-[2-(4-oxo-3,4-dihydroquinazolin-3-yl)ethoxy]phenyl}propanamide;
N1,2-Dimethyl-3-{4-[2-(4-oxo-3,4-dihydroquinazolin-3-yl)ethoxy]phenyl}-2-ethoxypropanamide;
Methyl 2-ethoxy-3-{4-[2-(4-oxo-3,4-dihydro-quinazolin-3-yl)ethoxy]phenyl}propanoate;
Ethyl 2-isopropylthio-3-{4-[2-(4-oxo-3,4-dihydro-quinazolin-3-yl)ethoxy]phenyl}propanoate;
Ethyl 2-ethylamino-3-{4-[2-(4-oxo-3,4-dihydro-quinazolin-3-yl)ethoxy]phenyl}propanoate;
2-Ethoxy-3-{4-[2-(4-oxo-3,4-dihydroquinazolin-3-yl)-ethoxy]phenyl}propanoic acid;
2-Ethoxy-3-{4-[3-(4-oxo-3,4-dihydroquinazolin-3-yl)-propyl]phenyl}propanoic acid;
2-Isopropylthio-3-{4-[2-(4-oxo-3,4-dihydro-quinazolin-3-yl)ethoxy]phenyl}propanoic acid;
2-Ethylamino-3-{4-[2-(4-oxo-3,4-dihydroquinazolin-3-yl)ethoxy]phenyl}propanoic acid;
2-Methyl-2-propoxy-3-{4-[3-(4-oxo-3,4-dihydro-quinazolin-3-yl)propyl]phenyl}propanoic acid;
2-Isopropoxy-3-{4-[2-(1-oxo-1,2-dihydro-isoquinolin-2-yl)ethoxy]phenyl}propanamide;
2-Phenoxy-3-{4-[2-(1-oxo-1,2-dihydroisoquinolin-2-yl)ethoxy]phenyl}propanamide;
2-Methyl-2-propoxy-3-{4-[3-(1-oxo-1,2-dihydro-isoquinolin-2-yl)propyl]phenyl}propanamide;
2-Isopropylthio-3-{4-[2-(1-oxo-1,2-dihydro-isoquinolin-2-yl)ethoxy]phenyl}propanamide;
2-Ethylamino-3-{4-[2-(1-oxo-1,2-dihydro-isoquinolin-2-yl)ethoxy]phenyl}propanamide;
N1,2-Dimethyl-3-(4-[2-(1-oxo-1,2-dihydro-isoquinolin-2-yl)ethoxy]phenyl}-2-ethoxypropanamide;
Methyl 2-ethoxy-3-{4-[2-(1-oxo-1,2-dihydro-isoquinolin-2-yl)ethoxy]phenyl}propanoate;
Ethyl 2-isopropylthio-3-{4-[2-(1-oxo-1,2-dihydro-isoquinolin-2-yl)ethoxy]phenyl}propanoate;
2-Ethoxy-3-{4-[3-(1-oxo-1,2-dihydroisoquinolin-2-yl)propyl]phenyl}propanoic acid;
2-Isopropylthio-3-{4-[2-(1-oxo-1,2-dihydro-isoquinolin-2-yl)ethoxy]phenyl}propanoic acid;
2-Ethylamino-3-{4-[2-(1-oxo-1,2-dihydro-isoquinolin-2-yl)ethoxy]phenyl}propanoic acid;
2-Methyl-2-propoxy-3-{4-[3-(1-oxo-1,2-dihydro-isoquinolin-2-yl)propyl]phenyl}propanoic acid;
2-Ethoxy-3-{4-[2-(4-oxo-3,4-dihydro-1,2,3-benztriazin-3-yl)ethoxy]phenyl}propanamide;
2-Ethoxy-3-{4-[3-(4-oxo-3,4-dihydro-1,2,3-benztriazin-3-yl)propyl]phenyl}propanamide;
2-Isopropoxy-3-{4-[3-(4-oxo-3,4-dihydro-1,2,3-benztriazin-3-yl)propyl]phenyl}propanamide;
2-Ethylthio-3-{4-[2-(4-oxo-3,4-dihydro-1,2,3-benztriazin-3-yl)ethoxy]phenyl}propanamide;
2-Ethylamino-3-{4-[2-(4-oxo-3,4-dihydro-1,2,3-benztriazin-3-yl)ethoxy]phenyl}propanamide;
2-Ethoxy-2-methyl-3-{4-[2-(4-oxo-3,4-dihydro-1,2,3-benztriazin-3-yl)ethoxy]phenyl}propanamide;
N1,2-Dimethyl-3-{4-[2-(4-oxo-3,4-dihydro-1,2,3-benztriazin-3-yl)ethoxy]phenyl}-2-ethoxypropanamide;
Methyl 2-ethoxy-3-{4-[2-(4-oxo-3,4-dihydro-1,2,3-benztriazin-3-yl)ethoxy]phenyl}propanoate;
Ethyl 2-ethylthio-3-{4-[2-(4-oxo-3,4-dihydro-1,2,3-benztriazin-3-yl)ethoxy]phenyl}propanoate;
Ethyl 2-ethoxy-2-methyl-3-(4-[2-(4-oxo-3,4-dihydro-1,2,3-benztriazin-3-yl)ethoxy]phenyl}propanoate;
2-Ethoxy-3-{4-[2-(4-oxo-3,4-dihydro-1,2,3-benztriazin-3-yl)ethoxy]phenyl}propanoic acid;
2-Ethoxy-3-{4-[3-(4-oxo-3,4-dihydro-1,2,3-benztriazin-3-yl)propyl]phenyl}propanoic acid;
2-Isopropoxy-3-{4-[3-(4-oxo-3,4-dihydro-1,2,3-benztriazin-3-yl)propyl]phenyl}propanoic acid;
2-Ethylthio-3-{4-[2-(4-oxo-3,4-dihydro-1,2,3-benztriazin-3-yl)ethoxy]phenyl}propanoic acid;
2-Ethylamino-3-{4-[2-(4-oxo-3,4-dihydro-1,2,3-benztriazin-3-yl)ethoxy]phenyl}propanoic acid;
2-Ethoxy-2-methyl-3-{4-[2-(4-oxo-3,4-dihydro-1,2,3-benztriazin-3-yl)ethoxy]phenyl}propanoic acid;
2-Isopropylthio-3-{4-[2-(1-oxo-1,2-dihydro-phthalazin-2-yl)ethoxy]phenyl}propanohydroxamic acid;
2-Ethylamino-3-{4-[2-(1-oxo-1,2-dihydrophthalazin-2-yl)ethoxy]phenyl}propanohydroxamic acid;
2-Propoxy-3-{4-[2-(4-oxo-3,4-dihydro-2H-1,3-benzoxazin-3-yl)ethoxy]phenyl}propanohydroxamic acid;
2-Propylthio-3-{4-[2-(4-oxo-3,4-dihydro-2H-1,3-benzoxazin-3-yl)ethoxy]phenyl}propanohydroxamic acid;
2-Ethoxy-3-(4-[2-(4-oxo-1,2,3,4-tetrahydro-quinazolin-3-yl)ethoxy]phenyl}propanohydroxamic acid;
2-Propoxy-3-{4-[2-(4-oxo-3,4-dihydro-2H-1,3-benzthiazin-3-yl)ethoxy]phenyl}propanohydroxamic acid;
2-Ethoxy-3-{4-[2-(1-oxo-1,2,3,4-tetrahydro-isoquinolin-2-yl)ethoxy]phenyl}propanohydroxamic acid;
2-Isopropoxy-3-{4-[2-(4-oxo-3,4-dihydroquinazolin-3-yl)ethoxy]phenyl}propanohydroxamic acid;
2-Ethoxycarbonyl-2-methoxy-3-{4-[2-(1-oxo-1,2-dihydrophthalazin-2-yl)ethoxy]phenyl}propanamide;
N-Methyl-2-ethoxy-2-ethoxycarbonyl-3-{4-[2-(1-oxo-1,2-dihydrophthalazin-2-yl)ethoxy]phenyl}propanamide;
2-Propoxy-3-{4-[2-(4-oxo-3,4-dihydro-2H-1,3-benzoxazin-3-yl)ethoxy]benzyl}malonamide;
Dimethyl 2-methoxy-3-{4-[2-(1-oxo-1,2-dihydro-phthalazin-2-yl)ethoxy]benzyl}malonate;
Diethyl 2-propylthio-3-{4-[2-(4-oxo-3,4-dihydro-2H-1,3-benzoxazin-3-yl)ethoxy]benzyl}malonate;
Diethyl 2-ethoxy-3-{4-[2-(4-oxo-3,4-dihydro-2H-1,3-benzoxazin-3-yl)ethoxy]benzyl}malonate;
2-Ethoxycarbonyl-2-methoxy-3-{4-[2-(4-oxo-3,4-dihydro-2H-1,3-benzoxazin-3-yl)ethoxy]phenyl}propanamide;
N-Methyl-2-ethoxy-2-ethoxycarbonyl-3-{4-[2-(4-oxo-3,4-dihydro-2H-1,3-benzoxazin-3-yl)ethoxy]phenyl}-propanamide;
2-Ethoxycarbonyl-2-isopropoxy-3-{4-[2-(4-oxo-3,4-dihydroquinazolin-3-yl)ethoxy]phenyl}propanamide;
Diethyl 2-ethoxy-3-{4-[2-(4-oxo-3,4-dihydro-quinazolin-3-yl)ethoxy]benzyl}malonate;
2-Ethoxycarbonyl-2-methoxy-3-{4-[2-(4-oxo-3,4-dihydroquinazolin-3-yl)ethoxy]phenyl}propanamide;

2-Ethoxy-3-{4-[2-(4-oxo-3,4-dihydroquinazolin-3-yl)ethoxy]benzyl}malonamide;
N-Methyl-2-ethoxy-2-ethoxycarbonyl-3-{4-[2-(4-oxo-3,4-dihydroquinazolin-3-yl)ethoxy]phenyl}propanamide;
2-Ethoxycarbonyl-2-isopropoxy-3-{4-[2-(1-methyl-2,4-dioxo-1,2,3,4-tetrahydroquinazolin-3-yl)ethoxy]phenyl}-propanamide;
Diethyl 2-ethoxy-3-{4-[2-(1-methyl-2,4-dioxo-1,2,3,4-tetrahydroquinazolin-3-yl)ethoxy]benzyl}-malonate;
2-Ethoxy-3-{4-[2-(1-methyl-2,4-dioxo-1,2,3,4-tetrahydroquinazolin-3-yl)ethoxy]benzyl}malonamide; and
N-Methyl-2-ethoxy-2-ethoxycarbonyl-3-{4-[2-(1-methyl-2,4-dioxo-1,2,3,4-tetrahydroquinazolin-3-yl)ethoxy]phenyl}propanamide.

Test Example 1:

Antidiabetic effect on diabetic model mice:

The α-substituted phenylpropionic acid derivatives (1) according to the present invention were used to conduct a test for evaluating their activities against model mice suffering from non-insulin dependent diabetes mellitus (NIDDM). Incidentally, pioglitazone was used as a comparative compound.

(Testing method)

db/db Mice (Crea Co.; preliminarily rearing male mice aged 7 to 9 weeks for a week and using 4 or 5 mice having a blood glucose level of at least 300 mg/dl as a group) were used as subject animals. A predetermined amount of each of the compounds according to the present invention or the comparative compound was orally administered to the mice for 4 days once a day with the compound suspended in a 0.5% aqueous solution of sodium carboxymethyl cellulose. At 18 to 24 hours after the final administration, blood was collected from each of the subject animals to determine its blood glucose level (found value) by means of a simplified blood glucose meter, Mediace (manufactured by Terumo Corporation). On the other hand, the blood glucose level of a group of mice (control group) to which only a 0.5% aqueous solution of sodium carboxymethyl cellulose had been administered was also determined (control value).

On the basis of the thus-determined blood glucose level as to each of the compounds according to the present invention and the comparative compound, percent reduction of blood glucose was calculated out in accordance with the following equation. The results are shown in the following Table 21.

$$\text{Percent reduction (\%)} = [(\text{Control value} - \text{Found value})/(\text{Control value})] \times 100$$

Table 21

| Compound | Dose (mg/kg/day) | Percent reduction (%) | Compound | Dose (mg/kg/day) | Percent reduction (%) |
|---|---|---|---|---|---|
| 1A | 50 | 73 | 1W | 50 | 50 |
| 1B | 20 | 43 | 1X | 50 | 60 |
| 1J | 20 | 73 | 1AA | 20 | 48 |
| 1K | 50 | 69 | 1AR | 20 | 38 |
| 1N | 50 | 72 | 1BC | 10 | 50 |
| 1O | 50 | 42 | 1BI | 20 | 52 |
| 1P | 50 | 66 | 1BS | 20 | 60 |
| 1Q | 50 | 49 | Pioglitazone | 20 | 26 |
| 1T | 50 | 53 | Pioglitazone | 50 | 44 |

As apparent from the results shown in the table, the compounds according to the present invention have an excellent antidiabetic effect.

## Claims

1. An $\alpha$-substituted phenylpropionic acid derivative represented by the following formula (1):

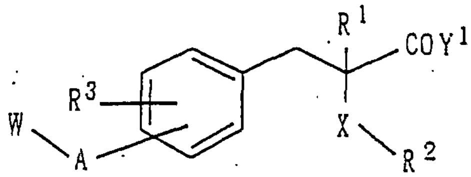

(1)

wherein A is a linear or branched $C_{1-8}$ alkylene group which may be substituted by 1-5 hydroxy groups, a linear or branched $C_{1-8}$ alkyleneoxy group which may be substituted by 1-5 hydroxy groups, or a linear or branched $C_{2-9}$ alkylenecarbonyl group which may be substituted by 1-5 hydroxy groups, X is O, S, NH or $CH_2$, $Y^1$ is an amino, hydroxyamino, hydroxy-$C_{1-6}$-alkylamino, mono-$C_{1-6}$-alkylamino, di-$C_{1-6}$-alkylamino, 4- to 7-membered cyclic amino, hydroxy or a linear or branched $C_{1-6}$-alkoxy group, $R^1$ is a hydrogen atom, a linear or branched $C_{1-6}$ alkyl group or $COY^2$ (in which $Y^2$ is an amino, hydroxyamino, hydroxy-$C_{1-6}$-alkylamino, mono-$C_{1-6}$-alkylamino, di-$C_{1-6}$-alkylamino, 4- to 7-membered cyclic amino, hydroxy or a linear or branched $C_{1-6}$-alkoxy group), $R^2$ is a linear or branched $C_{1-6}$ alkyl group, $C_{1-6}$-alkoxy-$C_{1-6}$-alkyl group, a hydroxy-$C_{1-6}$-alkyl group, halogeno-$C_{1-6}$-alkyl group having 1-3 halogen atoms, $COY^2$ (in which $Y^2$ has the same meaning as defined above), or a phenyl, pyridyl, phenyl-$C_{1-6}$-alkyl group, $R^3$ is a hydrogen or halogen atom, or a linear or branched $C_{1-8}$ alkyl group, a linear or branched $C_{1-8}$ alkoxy group, halogeno-$C_{1-8}$-alkyl group having 1-3 halogen atoms, amino, hydroxy, a $C_{1-9}$ alkanoyl group or benzoyl group, W is a bicyclic lactam ring selected from the following groups (W-1) to (W-6) and (W-9):

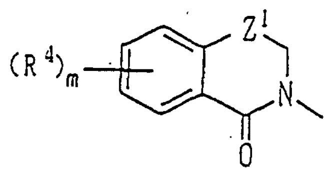

(W－1)

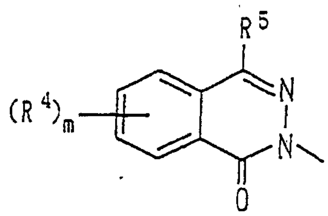

(W－2)

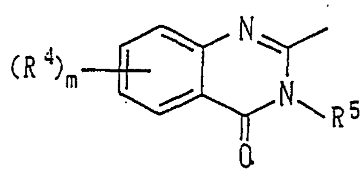

(W－3)

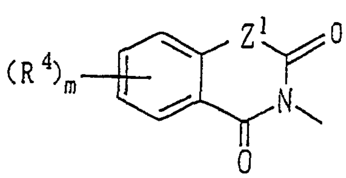

(W－4)

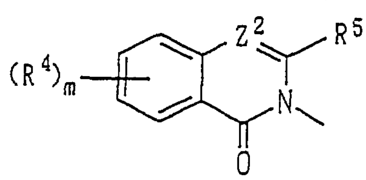

(W－5)

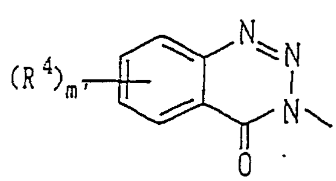

(W－6)

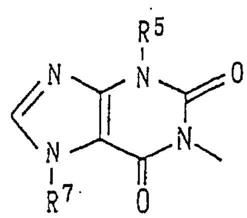

(W－9)

wherein $R^4$ is a hydrogen or halogen atom, a linear or branched $C_{1-8}$ alkyl group, a linear or branched $C_{1-8}$ alkoxy

group, halogeno-$C_{1-8}$-alkyl group having 1-3 halogen atoms, amino, hydroxy, cyano, carbamoyl, $C_{1-9}$ alkanoyl group, benzoyl, nitro, carboxy or sulfonamide group, or phenyl or benzyloxy, $R^5$ is a hydrogen atom, a linear or branched $C_{1-8}$ alkyl group, or $C_{6-14}$ aryl group, phenyl-$C_{1-6}$-alkyl group or pyridyl group, $R^7$ is a linear or branched $C_{1-6}$ alkyl group, phenyl or phenyl-$C_{1-6}$-alkyl group, $Z^1$ is O, S, $CH_2$ or $NR^5$ (in which $R^5$ has the same meaning as defined above), $Z^2$ is N or CH, and m is an integer of 1 to 4; or a salt thereof.

**2.** An α-substituted phenylpropionic acid derivative represented by anyone of the following formulas (1AJ), (1AK), (1AL), (1AO), (1AQ), (1AS), (1AU) and (1AV), or a salt thereof:

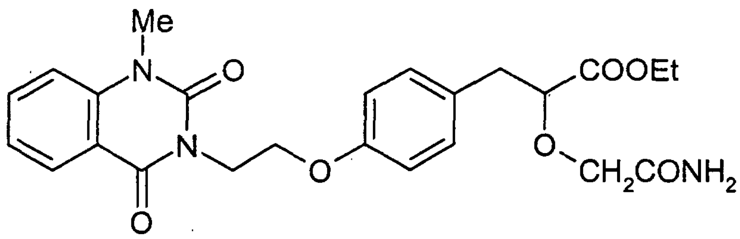

(1AJ),

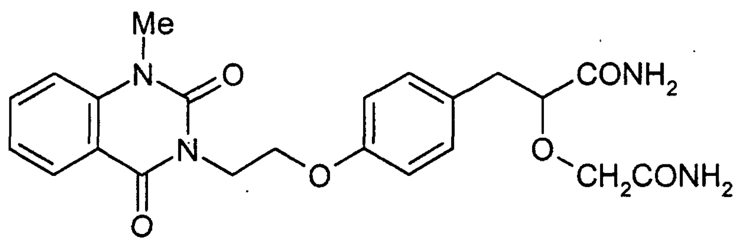

(1AK),

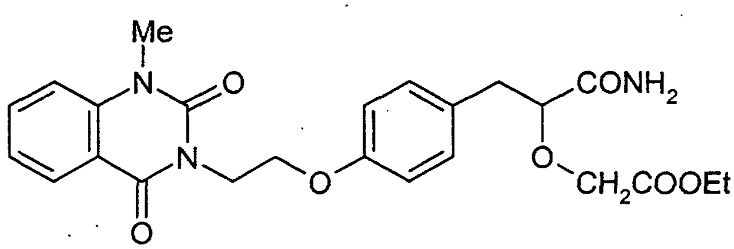

(1AL),

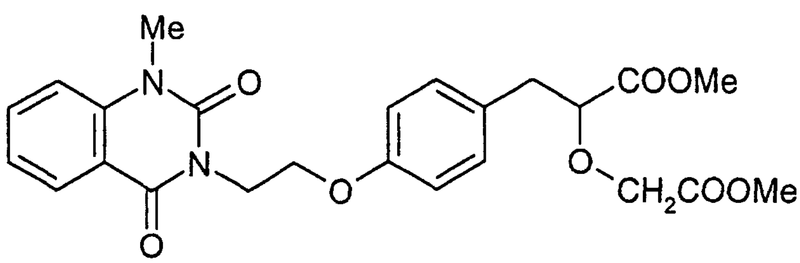

(1AO),

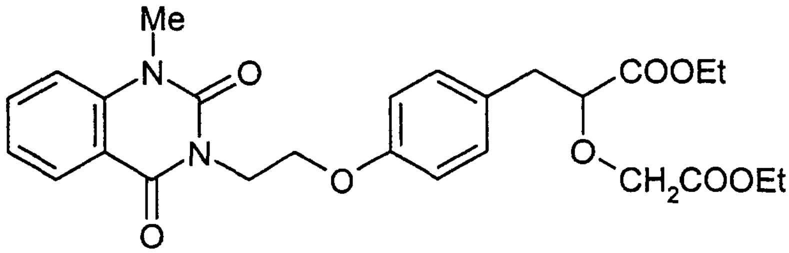

(1AQ),

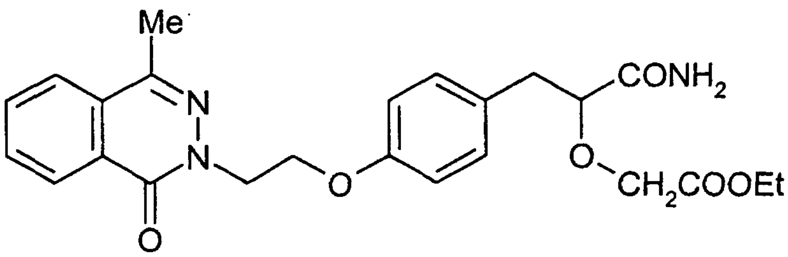

(1AS),

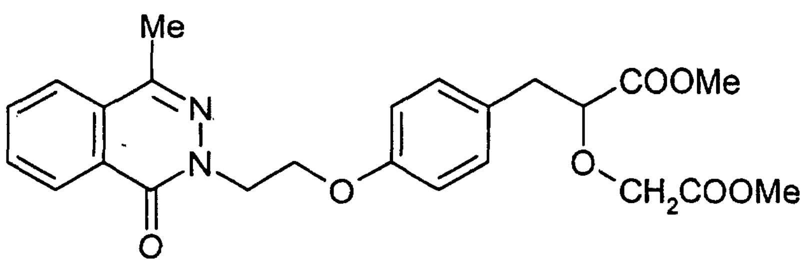

(1AU),

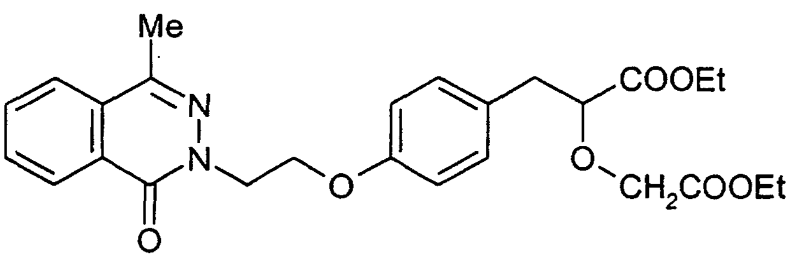

(1AV).

3. A medicine comprising the α-substituted phenylpropionic acid derivative or the salt thereof according to Claim 1 or 2 as an active ingredient.

4. The medicine according to Claim 3, which is an antidiabetic.

5. The medicine according to Claim 4, which is an agent for lowering lipid.

**6.** A medicinal composition comprising the α-substituted phenylpropionic acid derivative or the salt thereof according to Claim 1 or 2 and a pharmaceutically acceptable carrier.

**7.** Use of the α-substituted phenylpropionic acid derivative or the salt thereof according to Claim 1 or 2 for the manufacture of a medicine.

**Patentansprüche**

**1.** α-Substituiertes Phenylpropionsäurederivat, wiedergegeben durch die nachstehende Formel (1):

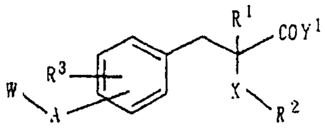
(1)

worin A eine lineare oder verzweigte $C_{1-8}$-Alkylengruppe, die mit 1-5 Hydroxygruppen substituiert sein kann, eine lineare oder verzweigte $C_{1-8}$-Alkylenoxygruppe, die mit 1-5 Hydroxygruppen substituiert sein kann, oder eine lineare oder verzweigte $C_{2-9}$-Alkylencarbonylgruppe, die mit 1-5 Hydroxygruppen substituiert sein kann, darstellt; X O, S, NH oder $CH_2$ darstellt, $Y^1$ eine Amino-, Hydroxyamino-, Hydroxy-$C_{1-6}$-alkylamino-, Mono-$C_{1-6}$-alkylamino-, Di-$C_{1-6}$-alkylamino-, 4- bis 7-gliedrige cyclische Amino-, Hydroxy- oder eine lineare oder verzweigte $C_{1-6}$-Alkoxygruppe darstellt, $R^1$ ein Wasserstoffatom, eine lineare oder verzweigte $C_{1-6}$-Alkylgruppe oder $COY^2$ (worin $Y^2$ eine Amino-, Hydroxyamino-, Hydroxy-$C_{1-6}$-alkylamino-, Mono-$C_{1-6}$alkylamino-, Di-$C_{1-6}$-alkylamino-, 4- bis 7-gliedrige cyclische Amino-, Hydroxy- oder eine lineare oder verzweigte $C_{1-6}$-Alkoxygruppe darstellt) darstellt; $R^2$ eine lineare oder verzweigte $C_{1-6}$-Alkylgruppe, $C_{1-6}$-Alkoxy-$C_{1-6}$-alkylgruppe, eine Hydroxy-$C_{1-6}$-alkylgruppe, Halogen-$C_{1-6}$-alkylgruppe mit 1-3 Halogenatomen, $COY^2$ (worin $Y^2$ die gleiche wie vorstehend definierte Bedeutung aufweist), oder eine Phenyl-, Pyridyl-, Phenyl-$C_{1-6}$-alkylgruppe darstellt; $R^3$ ein Wasserstoff- oder Halogenatom oder eine lineare oder verzweigte $C_{1-8}$-Alkylgruppe, eine lineare oder verzweigte $C_{1-8}$-Alkoxygruppe, Halogen-$C_{1-8}$-alkylgruppe mit 1-3 Halogenatomen, Amino-, Hydroxy-, eine $C_{1-9}$-Alkanoylgruppe oder eine Benzoylgruppe darstellt, W einen bicyclischen Lactamring darstellt, ausgewählt aus den nachstehenden Gruppen (W-1) bis (W-6) und (W-9):

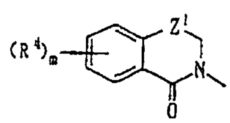
(W-1)

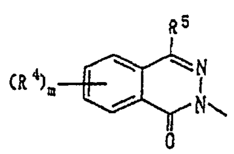
(W-2)

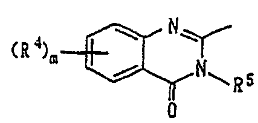
(W-3)

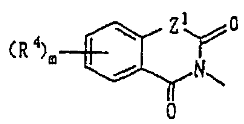
(W-4)

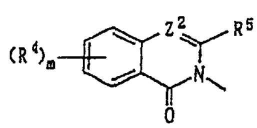
(W-5)

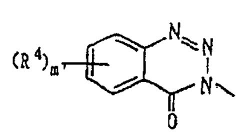
(W-6)

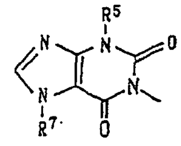
(W-9)

worin $R^4$ ein Wasserstoff- oder Halogenatom, eine lineare oder verzweigte $C_{1-8}$-Alkylgruppe, eine lineare oder verzweigte $C_{1-8}$-Alkoxygruppe, Halogen-$C_{1-8}$-alkylgruppe mit 1-3 Halogenatomen, Amino-, Hydroxy-, Cyano-, Carbamoyl-, $C_{1-9}$-Alkanoylgruppe, Benzoyl-, Nitro-, Carboxy- oder Sulfonamidgruppe, oder Phenyl oder Benzyloxy darstellt; $R^5$ ein Wasserstoffatom, eine lineare oder verzweigte $C_{1-8}$-Alkylgruppe oder $C_{6-14}$-Arylgruppe, Phenyl-$C_{1-6}$-alkylgruppe oder Pyridylgruppe darstellt; $R^7$ eine lineare oder verzweigte $C_{1-6}$-Alkylgruppe, Phenyl- oder

Phenyl-$C_{1-6}$-alkylgruppe darstellt, $Z^1$ O, S, $CH_2$ oder $NR^5$ (worin $R^5$ die gleiche wie vorstehend definierte Bedeutung aufweist) darstellt, $Z^2$ N oder CH darstellt und m eine ganze Zahl von 1 bis 4 ist, oder ein Salz davon.

**2.** α-Substituiertes Phenylpropionsäurederivat, wiedergegeben durch eine der nachstehenden Formeln (1AJ), (1AK), (1AL), (1AO), (1AQ), (1AS), (1AU) und (1AV) oder ein Salz davon:

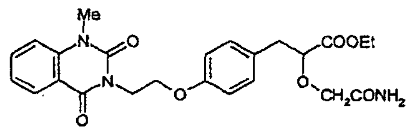

(1AJ),

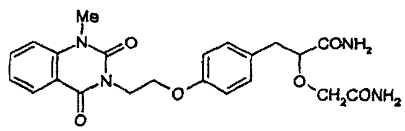

(1AK),

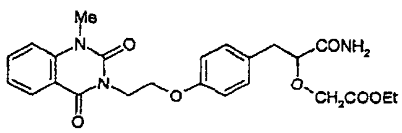

(1AL),

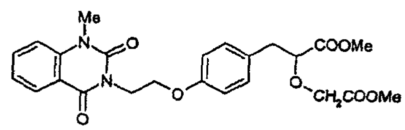

(1AO),

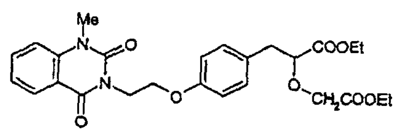

(1AQ),

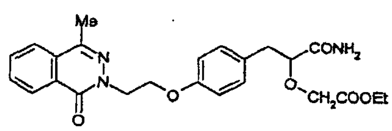

(1AS),

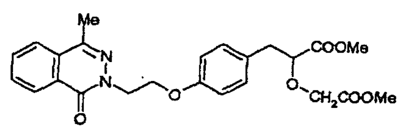

(1AU),

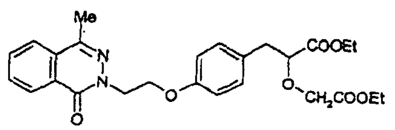

(1AV).

3. Arzneimittel, umfassend das α-substituierte Phenylpropionsäurederivat oder das Salz davon nach Anspruch 1 oder 2 als einen Wirkstoff.

4. Arzneimittel nach Anspruch 3, welches ein Antidiabetikum darstellt.

5. Arzneimittel nach Anspruch 4, welches ein Mittel zur Lipidsenkung darstellt.

6. Arzneimittelzusammensetzung, umfassend das α-substituierte Phenylpropionsäurederivat oder das Salz davon nach Anspruch 1 oder 2 und einen pharmazeutisch verträglichen Träger.

7. Verwendung des α-substituierten Phenylpropionsäurederivats oder des Salzes davon nach Anspruch 1 oder 2 zur Herstellung eines Arzneimittels.

**Revendications**

1. Dérivé d'acide phénylpropionique α-substitué représenté par la formule (1) suivante :

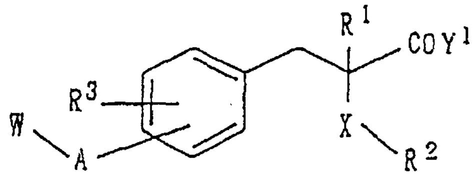

(1)

dans laquelle A est un groupe alkylène en $C_{1-8}$ linéaire ou ramifié qui peut être substitué par 1 à 5 groupe(s) hydroxy, un groupe alkylèneoxy en $C_{1-8}$ linéaire ou ramifié qui peut être substitué par 1 à 5 groupe(s) hydroxy, ou un groupe alkylènecarbonyle en $C_{2-9}$ linéaire ou ramifié qui peut être substitué par 1 à 5 groupe(s) hydroxy, X est O, S, NH ou $CH_2$, $Y^1$ est un groupe amino, hydroxyamino, hydroxy-alkylamino en $C_{1-6}$, monoalkylamino en $C_{1-6}$, di(alkyl en $C_{1-6}$) amino, amino cyclique à 4 - 7 chaînons, hydroxy ou alkoxy en $C_{1-6}$ linéaire ou ramifié, $R^1$ est un atome d'hydrogène, un groupe alkyle en $C_{1-6}$ linéaire ou ramifié ou $COY^2$ (où $Y^2$ est un groupe amino, hydroxyamino, hydroxy-alkylamino en $C_{1-6}$, monoalkylamino en $C_{1-6}$, di(alkyl en $C_{1-6}$) amino, amino cyclique à 4 - 7 chaînons, hydroxy ou alkoxy en $C_{1-6}$ linéaire ou ramifié), $R^2$ est un groupe alkyle en $C_{1-6}$ linéaire ou ramifié, un groupe alkoxy en $C_{1-6}$-alkyle en $C_{1-6}$, un groupe hydroxy-alkyle en $C_{1-6}$, un groupe halogéno-alkyle en $C_{1-6}$ ayant de 1 à 3 atome(s) d'halogène, $COY^2$ (où $Y^2$ a la même signification que définie ci-dessus), ou un groupe phényle, pyridyle, phényl-alkyle en $C_{1-6}$, $R^3$ est un atome d'hydrogène ou d'halogène, ou un groupe alkyle en $C_{1-8}$ linéaire ou ramifié, un groupe alkoxy en $C_{1-8}$ linéaire ou ramifié, un groupe halogéno-alkyle en $C_{1-8}$ ayant de 1 à 3 atome(s) d'halogène, amino, hydroxy, un groupe alcanoyle en $C_{1-9}$ ou un groupe benzoyle, W est un groupe lactame bicyclique choisi parmi les groupes suivants (W-1) à (W-6) et (W-9) :

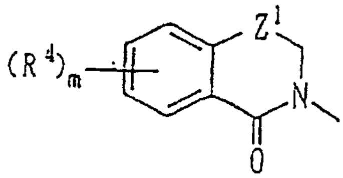

(W − 1)

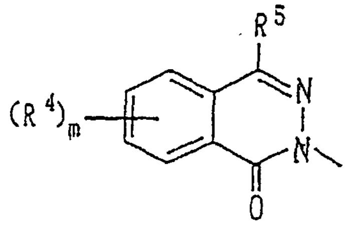

(W − 2)

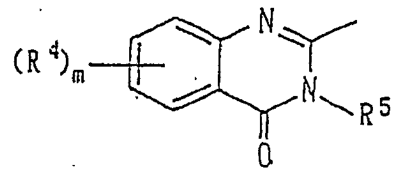

(W − 3)

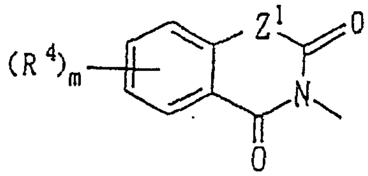

(W − 4)

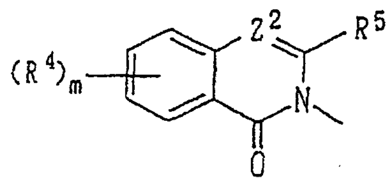

(W − 5)

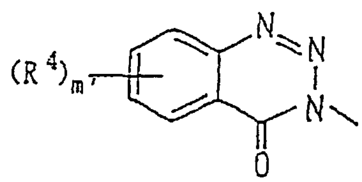

(W − 6)

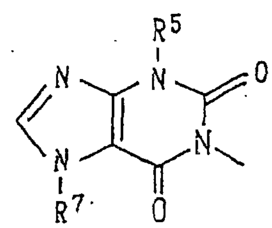

(W − 9)

dans lesquels $R^4$ est un atome d'hydrogène ou d'halogène, un groupe alkyle en $C_{1-8}$ linéaire ou ramifié, un groupe alkoxy en $C_{1-8}$ linéaire ou ramifié, un groupe halogéno-alkyle en $C_{1-8}$ ayant de 1 à 3 atome(s) d'halogène, un groupe amino, hydroxy, cyano, carbamoyle, alcanoyle en $C_{1-9}$, benzoyle, nitro, carboxy ou sulfonamide, ou phényle ou benzyloxy, $R^5$ est un atome d'hydrogène, un groupe alkyle en $C_{1-8}$ linéaire ou ramifié ou un groupe aryle en $C_{6-14}$, un groupe phényl-alkyle en $C_{1-6}$ ou pyridyle, $R^7$ est un groupe alkyle en $C_{1-6}$ linéaire ou ramifié, phényle ou phényl-alkyle en $C_{1-6}$, $Z^1$ est O, S, $CH_2$ ou $NR^5$ (où $R^5$ a la même signification que ci-dessus), $Z^2$ est N ou CH, et m est un nombre entier de 1 à 4 ; ou sel de celui-ci.

**2.** Dérivé d'acide phénylpropionique α-substitué représenté par l'une quelconque des formules suivantes (1AJ), (1AK), (1AL), (1AO), (1AQ), (1AS), (1AU) et (1AV), ou sel de celui-ci :

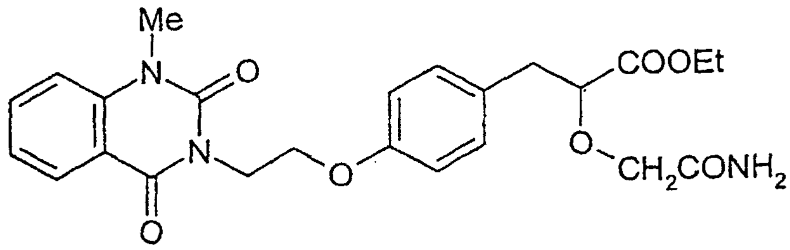

(1AJ),

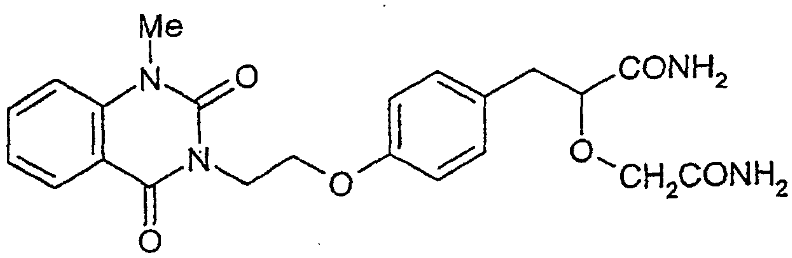

(1AK),

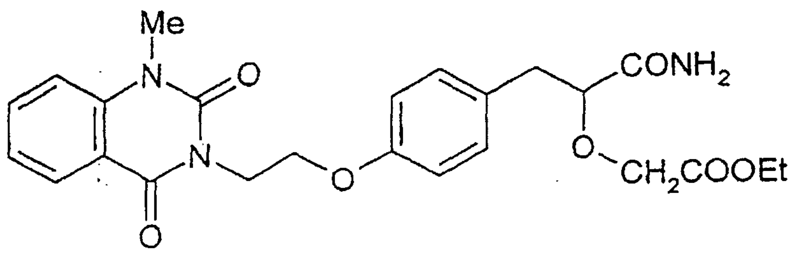

(1AL),

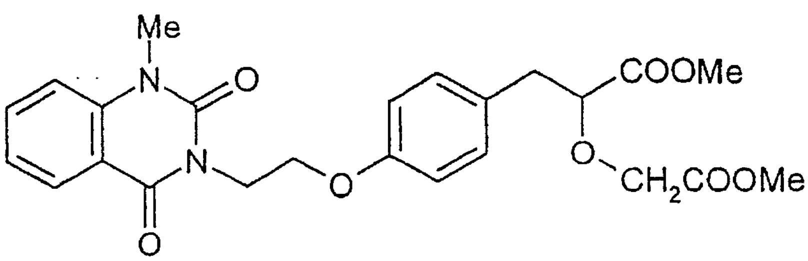

(1AO),

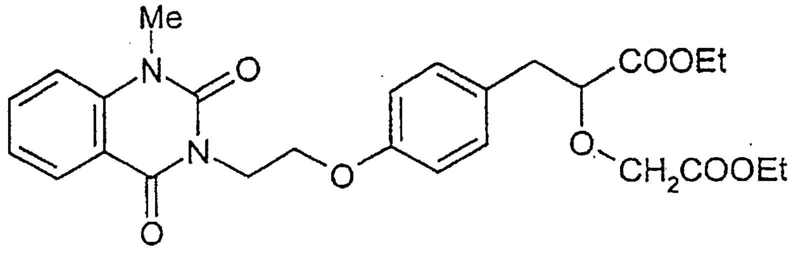

(1AQ),

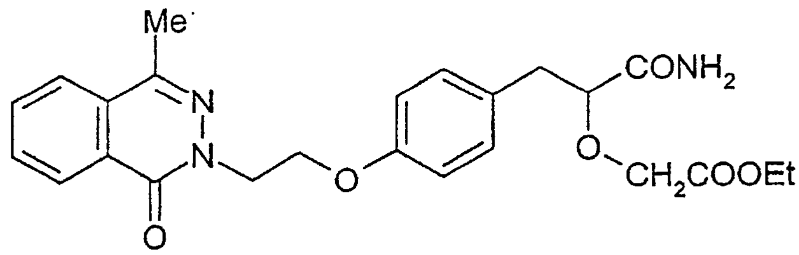

(1AS),

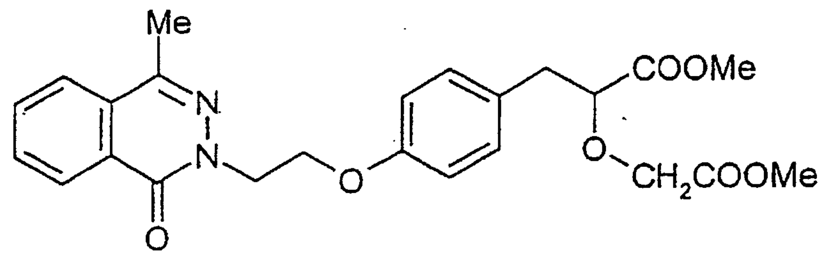

(1AU),

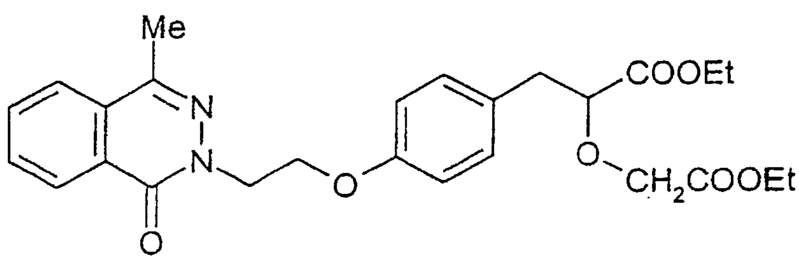

(1AV).

**3.** Médicament comprenant le dérivé d'acide phénylpropionique $\alpha$-substitué ou le sel de celui-ci selon la Revendication 1 ou 2 en tant qu'ingrédient actif.

**4.** Médicament selon la Revendication 3, qui est un antidiabétique.

**5.** Médicament selon la Revendication 4, qui est un agent pour réduire les lipides.

**6.** Composition médicinale comprenant le dérivé d'acide phénylpropionique $\alpha$-substitué ou le sel de celui-ci selon la Revendication 1 ou 2 et un véhicule pharmaceutiquement acceptable.

**7.** Utilisation du dérivé d'acide phénylpropionique $\alpha$-substitué ou du sel de celui-ci selon la Revendication 1 ou 2 pour la fabrication d'un médicament.